(19) Europäisches Patentamt
European Patent Office
Office européen des brevets

(11) Veröffentlichungsnummer : **0 549 532 A1**

(12) **EUROPÄISCHE PATENTANMELDUNG**

(21) Anmeldenummer : **92810995.8**

(22) Anmeldetag : **11.12.92**

(51) Int. Cl.[5] : **C07D 401/12,** C07D 403/12, A01N 43/647

(30) Priorität : **20.12.91 CH 3812/91**

(43) Veröffentlichungstag der Anmeldung : **30.06.93 Patentblatt 93/26**

(84) Benannte Vertragsstaaten : **AT BE CH DE DK ES FR GB GR IE IT LI LU MC NL PT SE**

(71) Anmelder : **CIBA-GEIGY AG**
**Klybeckstrasse 141**
**CH-4002 Basel (CH)**

(72) Erfinder : **Sutter, Marius, Dr.**
**Margarethenstrasse 75**
**CH-4102 Binningen (CH)**
Erfinder : **Müller, Urs, Dr.**
**Drosselstrasse 6**
**CH-4142 Münchenstein (CH)**
Erfinder : **Hostettler, Bernhard, Dr.**
**Im Obstgarten 7**
**CH-8044 Zürich (CH)**
Erfinder : **Ackermann, Peter**
**Hangelimattweg 113**
**CH-4148 Pfeffingen (CH)**
Erfinder : **Yamaguchi, Yasuchika, Dr.**
**Haltingerstrasse 75**
**CH-4057 Basel (CH)**

(54) **Benztriazolsulfonsäure-Derivate und ihre Verwendung als Mikrobizide.**

(57) Benztriazolsulfonsäure-Derivat der Formel I

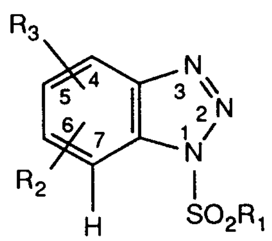

(I)

worin die $R_3 = R_4X$ ist und $R_4$ einen ungesättigten 6-gliedrigen Heterocyclus mit maximal zwei N-Atomen bedeutet, wobei der Heterocyclus substituiert sein kann, und X Sauerstoff oder Schwefel darstellt, währen $R_1$ und $R_2$ die hierin genannten Bedeutungen haben, stellen wertvolle Mikrobizide dar. Sie können im Pflanzenschutz in Form geeigneter Mittel beispielsweise zur Bekämpfung von Pilzkrankheiten eingesetzt werden.

EP 0 549 532 A1

Die vorliegende Erfindung betrifft neue Benztriazolsulfonsäure-Derivate der nachstehenden Formel I. Sie betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung oder Verhütung eines Befalls von Pflanzen durch phytopathogene Mikroorganismen, vorzugsweise Fungi.

Die erfindungsgemässen Verbindungen entsprechen der allgemeinen Formel I

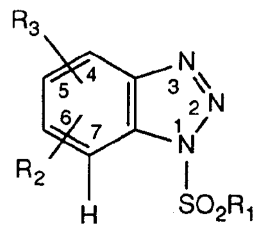

(I)

worin die $R_1SO_2$-Gruppe die 1- oder 3-Position besetzt und im Verhältnis zu den Substituenten $R_3$ und $R_2$ reine oder gemischte Stellungsisomere bildet, und worin die Substituenten folgende Bedeutung haben:

$R_3$ = $R_4X$

X = Sauerstoff oder Schwefel;

$R_4$ = ungesättigter 6-gliedriger Heterocyclus mit maximal zwei N-Atomen, der unsubstituiert oder durch mindestens einen der Substituenten Halogen, $C_1$-$C_6$-Alkyl, $C_1$-$C_6$-Alkoxy, $C_1$-$C_6$-Alkylthio, $C_1$-$C_6$-Haloalkyl, $C_1$-$C_6$-Haloalkoxy, $C_3$-$C_6$-Cycloalkyl, Cyano, Nitro, -COO($C_1$-$C_6$-Alkyl) und N(R')(R") substituiert sein kann;

$R_2$ = Wasserstoff, Halogen, $C_1$-$C_4$-Alkyl, $C_1$-$C_4$-Alkoxy, $C_1$-$C_4$-Haloalkyl, $C_1$-$C_4$-Haloalkoxy und $-N(alk)_2$;

$R_1$ = $C_1$-$C_4$-Alkyl, $C_1$-$C_4$-Haloalkyl, $C_3$-$C_6$-Cycloalkyl, oder $-N(alk)_2$, wobei alk $C_1$-$C_4$-Alkyl bedeutet und in gleicher oder verschiedener Bedeutung an N gebunden ist;

R' und R" = unabhängig Wasserstoff oder $C_1$-$C_4$-Alkyl.

Unter dem Begriff Alkyl selbst oder als Bestandteil eines anderen Substituenten wie Haloalkyl, Alkoxy oder Haloalkoxy, sind je nach Zahl der angegebenen Kohlenstoffatome beispielsweise die folgenden geradkettigen oder verzweigten Gruppen zu verstehen: Methyl, Ethyl, Propyl, Butyl, Pentyl, Hexyl sowie ihre Isomeren, Isopropyl, Isobutyl, sek. Butyl, sek. Amyl, tert. Amyl, tert.-Butyl. Halogen und Halo stehen für Fluor, Chlor, Brom oder Jod. Haloalkoxy steht somit für einen einfach bis perhalogenierten Alkoxyrest, wie z.B. $OCH_2F$, $OCHF_2$, $OCHFCH_3$, $OCH_2CH_2Br$, $OCF_2CHFCl$ usw.

Der Begriff Cycloalkyl steht für Cyclopropyl, Cyclobutyl, Cyclopentyl oder Cyclohexyl.

Unter den angegebenen ungesättigten 6-gliedrigen Heterocyclen sind Pyridin, Pyrimidin, Pyrazin und Pyridazin zu verstehen.

Die Verbindungen der Formel I sind bei Raumtemperatur stabile Oele oder Feststoffe, die sich durch wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen. Die erfindungsgemässen Wirkstoffe der Formel I zeichnen sich bei niedrigen Anwendungskonzentrationen nicht nur durch hervorragende mikrobizide, insbesondere fungizide Wirkung, sondern auch durch besonders gute Pflanzenverträglichkeit aus.

Im Umfang der Formel I sind jene Verbindungen von Wichtigkeit, worin $R_4$ einen substituierten Pyridin-, Pyrimidin-, Pyrazin- oder Pyridazinring bedeutet [Untergruppe Ib], und unter diesen solche, worin der 6-gliedrige Heterocyclus durch ein bis drei Substituenten, ausgewählt aus Halogen, Methyl, Ethyl, Isopropyl, Methoxy, $C_1$-$C_2$-Haloalkyl mit F und/oder Cl als Halogen, $CF_3O$, $CHF_2O$, Cyclopropyl, Nitro, substituiert ist [Untergruppe IC]. Unter den wichtigen Stoffgruppen im Umfang der Formel I sind solche zu nennen, worin der 6-gliedrige Heterocyclus durch ein bis drei Substituenten, ausgewählt aus Halogen, Methyl, Ethyl, Isopropyl, Methoxy, $C_1$-$C_2$-Haloalkyl mit F und/oder Cl als Halogen, $CF_3O$, $CHF_2O$, Nitro, substituiert ist [Untergruppe Ic].

Eine der besonders wichtigen Stoffgruppen im Umfang der Formel I sind jene, worin der 6-gliedrige Heterocyclus unsubstituiertes oder ein- bis dreifach substituiertes Pyridin ist und $R_2$ Wasserstoff, Fluor, Chlor, Brom, Methyl, Methoxy, $CF_3$, $CF_3O$, $CHF_2O$ bedeutet [Untergruppe Id], besonders solche worin $R_2$ Wasserstoff Chlor oder Brom ist [Untergruppe Ie].

Eine der bevorzugten Verbindungsgruppen im Umfang der Untergruppe Id ist diejenige, worin der Pyridinring durch maximal drei Substituenten, ausgewählt aus Halogen, $C_1$-$C_4$-Alkyl, $C_1$-$C_4$-Alkoxy, $C_1$-$C_4$-Haloalkyl, $C_1$-$C_4$-Haloalkoxy und Cyclopropyl, substituiert ist [Untergruppe IF], und innerhalb dieser Gruppe besonders solche Verbindungen, worin der Pyridinring durch maximal drei Substituenten, ausgewählt aus Halogen,

$C_1$-$C_4$-Alkyl, $C_1$-$C_4$-Alkoxy, $C_1$-$C_4$-Haloalkyl und $C_1$-$C_4$-Haloalkoxy, substituiert ist [Untergruppe If], insbesondere jene, worin der Pyridinring mindestens durch $CF_3$ substituiert ist [Untergruppe Ig].

Eine andere bevorzugte Verbindungsgruppe im Umfang der Formel I sind solche, worin der 6-gliedrige Heterocyclus unsubstituiertes oder ein- bis dreifach substituiertes Pyrimidin ist und $R_2$ Wasserstoff, Fluor, Chlor, Brom, Methyl, Methoxy, $CF_3$, $CF_3O$ oder $CHF_2O$ bedeutet [Untergruppe Ih], insbesondere jene, worin $R_2$ Wasserstoff, Chlor oder Brom ist [Untergruppe Ii].

Innerhalb der Untergruppe Ih stellen solche Verbindungen eine wichtige Untergruppe dar, bei denen der Pyrimidinring durch maximal drei Substituenten ausgewählt aus Halogen, $C_1$-$C_4$-Alkyl, $C_1$-$C_4$-Alkoxy, $C_1$-$C_4$-Haloalkyl und $C_1$-$C_4$-Haloalkoxy substituiert ist, [Untergruppe Ij].

Ein bevorzugter Strukturtyp im Umfang der Formel I und ihrer hier genannten Untergruppen sind jene Verbindungen, bei denen $R_1$ Methyl ist. Ein anderer wichtiger Strukturtyp der Formel I und ihrer Untergruppen sind Benztriazol-Derivate, bei denen die 4-Position unsubstituiert ist.

Die Verbindungen der Formel I lassen sich herstellen

1) durch Reaktion einer Verbindung der Formel II

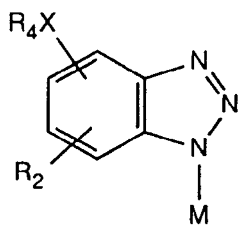

(II)

mit einer Verbindung der Formel III

$$Q\text{-}SO_2\text{-}R_1 \qquad \text{(III)}$$

worin X, $R_1$, $R_2$ und $R_4$ die unter Formel I angegebenen Bedeutungen besitzen und M Wasserstoff oder ein Alkalimetall, bevorzugt Na, K oder Li, und Q ein Halogenatom, bevorzugt Chlor, oder den Rest O-$SO_2$-$R_1$ darstellen, in einem inerten Lösungsmittel, gegebenenfalls in Gegenwart einer Base, bei Temperaturen von -30° bis 180°C, bevorzugt -10° bis 80°C, unter Normaldruck, vermindertem oder erhöhtem Druck, bevorzugt Normaldruck;

Befinden sich die Substituenten in Stellung 5 und/oder 6, so bilden sich zwei Isomere mit der Sulfonylgruppe in 1, bzw. 3 Stellung. Diese, wie auch beliebige Mischungen davon bilden auch einen Teil der Erfindung. Im folgenden ist jeweils nur eine dieser Strukturen gezeichnet, gemeint sind aber immer Gemische.

Verbindungen der Formel I lassen sich ferner herstellen

2) durch Diazotierung einer Verbindung der Formel IV

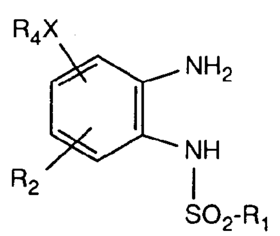

(IV)

worin X, $R_1$, $R_2$ und $R_4$ die unter Formel I angegebenen Bedeutungen besitzen, entweder

a) mit einem anorganischen Nitrit in einem Lösungsmittel, bevorzugt in Wasser oder in Gemischen von Wasser mit Alkoholen, bevorzugt Methanol oder Ethanol, oder in Gemischen von Wasser mit Ether, bevorzugt Dioxan oder Dimethoxyethan, in Gegenwart einer Säure bevorzugt Essigsäure bei Temperaturen von -30° bis 180°C, bevorzugt 0° bis 80°C; oder

b) mit einem organischen Nitrit, bevorzugt Ethylnitrit, Amylnitrit oder tert.Butylnitrit, in einem Lösungsmittel, bevorzugt Alkohole wie Methanol oder Ethanol oder Ether wie Dioxan oder Dimethoxyethan, gegebenenfalls in Gegenwart einer Säure, bei Temperaturen von -30° bis 180°C, bevorzugt 0° bis 80°C, unter Normaldruck oder erhöhtem Druck.

Die Verbindungen der Formel II mit M = H werden hergestellt

3) durch Diazotierung einer Verbindung der Formel V

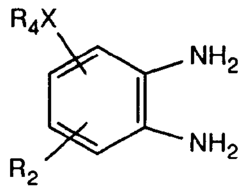

(V)

worin $R_4X$ und $R_2$ die unter Formel IIa angegebenen Bedeutungen besitzen, entweder

3a) mit einem anorganischen Nitrit in einem Lösungsmittel, bevorzugt in Wasser oder in Gemischen von Wasser mit Alkoholen, bevorzugt Methanol oder Ethanol, oder in Gemischen von Wasser mit Ether, bevorzugt Dioxan oder Dimethoxyethan, in Gegenwart einer Säure, bevorzugt Essigsäure, bei Temperaturen von -30° bis 180°C, bevorzugt 0° bis 80°C; oder

3b) mit einem organischen Nitrit, bevorzugt Ethylnitrit, Amylnitrit oder tert.Butylnitrit, in einem Lösungsmittel, bevorzugt Alkohole wie Methanol oder Ethanol oder Ether wie Dioxan oder Dimethoxyethan, gegebenenfalls in Gegenwart einer Säure bei Temperaturen von -30° bis 180°C, bevorzugt 0° bis 80°C, unter Normaldruck oder erhöhtem Druck; oder

4) durch Umsetzung einer Verbindung der Formel VI

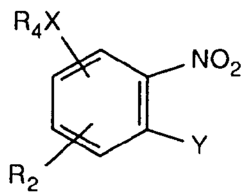

(VI),

worin $R_4X$ und $R_2$ die unter Formel IIa angegebenen Bedeutungen besitzen und Y ein Halogenatom, bevorzugt Fluor oder Chlor, darstellt mit Hydrazin oder Hydrazinhydrat in einem Lösungsmittel, bevorzugt Alkohole wie Methanol oder Ethanol, in Gegenwart eines säurebindenden Mittels, bevorzugt Natriumcarbonat, Kaliumcarbonat oder Triethylamin, bei Rückflusstemperaturen des Reaktionsmediums zu einer Verbindung der Formel VII

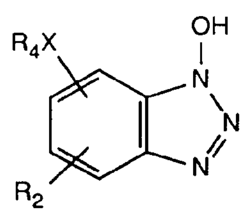

(VII),

welche anschliessend mit einem Chlorketon

$$Cl\text{-}CH_2\text{-}CO\text{-}(C_1\text{-}C_6\text{-Alkyl})$$

vorzugsweise Chloraceton, in einem inerten Lösungsmittel in Gegenwart einer Base bei Temperaturen von 40° bis 140°C, vorzugsweise 60° bis 120°C, zur Reaktion gebracht wird.

In den vorstehend beschriebenen Verfahren kommen folgende inerte Lösungsmittel in Frage: aliphatische, cycloaliphatische oder aromatische Kohlenwasserstoffe, wie z.B. Hexan, Cyclohexan, Toluol, Xylol, Petrolether oder Ligroin; chlorierte Kohlenwasserstoffe, wie z.B. Methylenchlorid, Chloroform, Tetrachlorkohlenstoff, Chlorbenzol; Ether, wie z.B. Diethylether, Diisopropylether, Furan, Tetrahydrofuran, Dioxan; Ketone, wie z.B Aceton, Methylethylketon; Alkohole, wie z.B. Methanol, Ethanol, Isopropanol; Ester, wie z.B. Essigsäureethylester, Essigsäurebutylester, Nitrile wie z.B. Acetonitril, Propionitril; Säureamide, wie z.B. Dimethylformamid; Sulfone und Sulfoxid, wie z.B. Dimethylsulfoxid, Sulfolan.

Als Basen oder säurebindende Mittel eignen sich z.B. Hydroxide, Carbonate, Hydrogencarbonate oder Alkoholate der Alkali- und Erdalkalimetalle; sowie tertiäre Amine, wie z.B. Triethylamin, Triisopropylamin, Pyridin oder 4-N,N-Dimethylaminopyridin.

Die vorstehend beschriebenen Verfahren entsprechen Synthesemethoden, die aus der Literatur bekannt sind. Sie sind z.B. beschrieben in Chem. Reviews 46, 1 (1950) und in der deutschen Offenlegungsschrift 34 06 011. Die Synthese von Ausgangsverbindungen des Typs der Formel IV ist in analoger Weise aus der US-Patentschrift 2,943,017 bekannt.

Verbindungen mit Benztriazolsulfonsäure-Strukturen sind bereits bekannt. Derartige Substanzen sind in den Patentschriften DE-1046937, GB-885,843 und US-2,943,017 als fungizid verwendbare Wirkstoffe beschrieben.

Verbindungen der Formel V lassen sich durch Reduktion der nitrogruppenhaltigen Verbindungen der Formel VIII herstellen. Als Reduktionsmittel können die klassischen Reduktionsmittel wie Eisen (Bechamps-Reduktion), Zinn(II)chlorid oder aber Wasserstoff mit einem Katalysator wie beispielsweise Raney-Nickel oder Palladium/Kohle verwendet werden. Die Reaktionsbedingungen entsprechen denen, die in der Literatur (z.B. Houben Weyl "Methoden der organischen Chemie") aufgeführt sind.

Verbindungen der Formel VIII lassen sich aus Verbindungen der Formel IX durch Umsetzung mit entsprechend substituierten 2-Halopyridinen, 2-Halopyrazinen, 3-Halopyridazinen, 2-Halopyrimidinen oder 4-Halopyrimidinen (Formelgruppe X) erhalten. Die Reaktion findet in inerten organischen Lösungsmitteln, bevorzugt in polaren wie DMF, DMSO, DMA oder Ketonen wie Aceton oder Ethylmethylketon, Alkoholen wie Ethanol, Propanol, Butanol oder Ethem wie Diethylether oder Tetrahydrofuran in Gegenwart einer Base, bevorzugt Alkali(hydrogen)carbonate oder -hydroxyde wie $Na_2CO_3$, $K_2CO_3$, NaOH oder KOH oder Amine wie Triethylamin oder Pyridin statt. Die Reaktionstemperatur liegt zwischen 0°C und + 150°C bevorzugt bei 80°-120°C.

Ein alternativer Zugang zu Verbindungen der Formel VIII ist die Umsetzung von Verbindungen der Formel XI mit entsprechend substituierten eine Hydroxy- oder Mercapto-Gruppe tragenden Pyridinen, Pyrimidinen, Pyridazinen oder Pyrazinen (Formelgruppe XII). Die Reaktion verläuft vorzugsweise unter denselben Bedingungen wie oben beschrieben.

Die Verbindungen der Formeln IX, X, XI und XII sind entweder in der Literatur bekannt, oder können nach bekannten Methoden hergestellt werden.

Im Falle von X = S können Verbindungen der Formel IX auch als Rhodanid maskiert eingesetzt werden (US-Patentschrift 4,076,828).

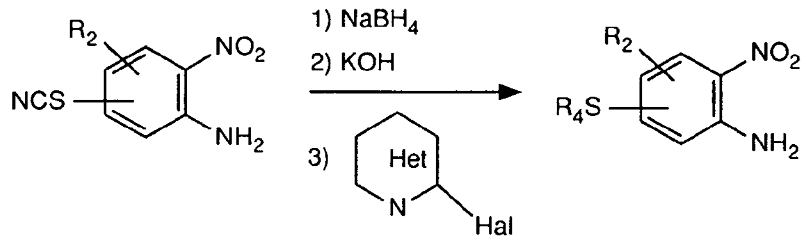


X

(Bedeutung von X siehe S.11)

Verbindungen der Formel II mit M = Wasserstoff lassen sich auch aus Verbindungen der Formel XIII durch Umsetzung mit 2-Halopyridinen, 2-Halopyrazinen, 3-Halopyridazinen oder 2-, bzw. 4-Halopyrimidinen (Formelgruppe X) synthetisieren. Die Reaktion wird in inerten organischen Lösungsmitteln, bevorzugt in polaren Lösungsmitteln wie beispielsweise DMF, DMSO, DMA oder Ketonen wie Aceton oder Ethylmethylketon, Alkoholen wie Ethanol, Propanol, Butanol oder Ethem wie Diethylether oder Tetrahydrofuran in Gegenwart einer Base durchgeführt. Bevorzugte Basen sind beispielsweise Carbonate wie Natriumcarbonat oder Kaliumcarbonat aber auch Hydroxyde wie Kaliumhydroxyd oder Natriumhydroxyd oder Amine wie Triethylamin oder Pyridin.Die Reaktionstemperatur liegt zwischen 0°C und 150°C bevorzugt bei 80°C bis 120°C.

Verbindungen der Formel XIII lassen sich aus Verbindungen der Formel XIV durch Diazotierung herstellen. Entweder

a) mit einem anorganischen Nitrit in einem Lösungsmittel, bevorzugt in Wasser oder in Gemischen von Wasser mit Alkoholen, bevorzugt Methanol oder Ethanol, oder in Gemischen von Wasser mit Ether, bevorzugt Dioxan oder Dimethoxyethan, in Gegenwart einer Säure bevorzugt Essigsäure bei Temperaturen von -30° bis 180°C, bevorzugt 0° bis 80°C; oder

b) mit einem organischen Nitrit, bevorzugt Ethylnitrit, Amylnitrit oder tert.Butylnitrit, in einem Lösungsmittel, bevorzugt Alkohole wie Methanol oder Ethanol oder Ether wie Dioxan oder Dimethoxyethan, gegebenenfalls in Gegenwart einer Säure, bei Temperaturen von -30° bis 180°C, bevorzugt 0° bis 80°C, unter Normaldruck oder erhöhtem Druck.

Verbindungen der Formel XIV lassen sich durch Reduktion der Nitrogruppe in Verbindungen der Formel IX herstellen. Als Reduktionsmittel können beispielsweise Eisen (Bechamps-Reduktion), Zinn(II)chlorid oder aber Wasserstoff in Gegenwart eines Katalysators wie z.B. Raney-Nickel oder Palladium auf Kohle verwendet werden. Die Reaktionsbedingungen entsprechen denen, die in der Literatur (z.B. Houben Weyl) angegeben

sind.

Verbindungen des Typs

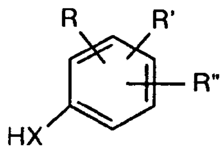

(IX, XIII und XIV)

können falls X = S ist, auch in der dimeren Form als Disulfid vorliegen. Die Mercaptoverbindungen können daraus durch Reduktion erhalten werden. Methoden sind aus der Literatur bekannt.

Allgemeines Reaktionsschema 1

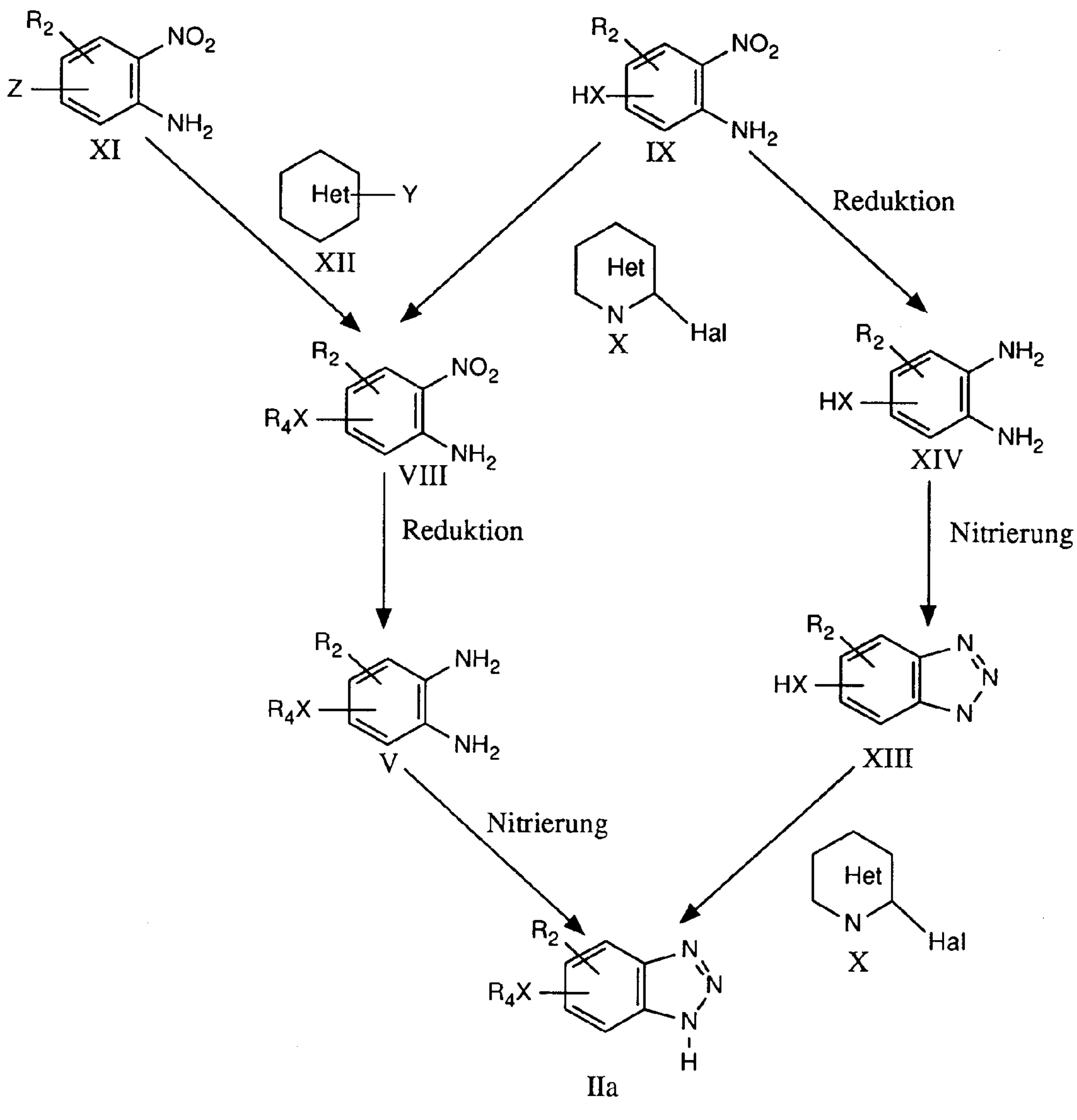

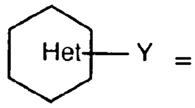

gegebenenfalls substituiertes Hydroxypyridin, Mercaptopyridin, Hydroxypyrimidin, Mercaptopyrimidin, Hydroxypyrazin, Mercaptopyrazin, Hydroxypyridazin oder Mercaptopyridazin

Z = Abgangsgruppe (wie z.B. Halogen), die sich o- oder p-ständig zu der $NO_2$-Gruppe befindet.

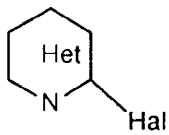

gegebenenfalls substituiertes 2-Halopyridin, -2-Halopyrazin, 2- oder 4-Halopyrimidin, 3-Halopyridazin

Verbindungen der Formel II mit M = H können auch analog zu den in der EP-A-355 049 für Verbindungen der Formel IIa mit $R_2$ = H und $R_3$ = subst. 2-Pyridyloxy beschriebenen Verfahren hergestellt werden. Verbindungen dieses Typs sind auch in der UK-2,157,679 sowie in der EP-A-299,446 beschrieben.

Falls $R_2$ Fluor, Chlor oder Brom ist, so können die entsprechenden Verbindungen auch durch Halogenierung von Verbindungen, in denen $R_2$ Wasserstoff bedeutet, erhalten werden. Dies gilt für Verbindungen der Formel II, V, VI, VII, VIII, IX, XI, XIII und XIV.

Reaktionsbedingungen für die Fluorierung, Chlorierung bzw. Bromierung sind in der chemischen Literatur (z.B. Houben Weyl "Methoden der organischen Chemie") gut bekannt.

Ueberraschenderweise wurde nun gefunden, dass Verbindungen der Formel I ein für praktische Bedürfnisse sehr günstiges biozides Spektrum zur Bekämpfung von phytopathogenen Mikroorganismen, insbesondere Fungi, aufweisen. Sie besitzen sehr vorteilhafte kurative und präventive Eigenschaften und werden zum Schutz von zahlreichen Kulturpflanzen eingesetzt. Mit den Wirkstoffen der Formel I können an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. vor phytopathogenen Pilzen verschont bleiben.

Die neuen Wirkstoffe der Formel I erweisen sich als bevorzugt wirksam gegen spezielle Gattungen der Pilzklasse Fungi imperfecti (z.B. Cercospora), Basidiomyceten (z.B. Puccinia) sowie Ascomyceten (z.B. Erysiphe und Venturia) und insbesondere gegen Oomyceten (z.B. Plasmopara, Peronospora, Pythium und Phytophthora). Sie stellen damit im Pflanzenschutz eine wertvolle Ergänzung der Mittel für die Bekämpfung von phytopathogenen Pilzen dar. Für ihre Anwendung in der Praxis besitzen sie vorteilhafterweise sowohl kurative wie auch präventive Eigenschaften und können zum Schutz von zahlreichen Kulturpflanzen eingesetzt werden. Mit diesen Wirkstoffen werden an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet, wobei auch später zuwachsende Pflanzenteile z.B. vor phytopathogenen Pilzen verschont bleiben. Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel I bzw. der neuen Mittel ausgezeichnet ist.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kem-, Stein und Beerenobst (Aepfel, Birnen, Pflaumen, Pfirsiche, Mandeln, Kirschen, Erd-, Him- und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen,

Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Verbindungen der Formel I können auch auf Samenkörner aufgebracht werden (Coating), indem man die Körner entweder in einer flüssigen Zubereitung des Wirkstoffs tränkt oder sie mit einer festen Zubereitung beschichtet.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Günstige Aufwandmengen liegen im allgemeinen bei 5 g bis 2 kg Aktivsubstanz (AS) je Hektar (ha), bevorzugt bei 10 g bis 1 kg AS/ha, insbesondere bei 20 g bis 600 g AS/ha.

Die Formulierungen, d. h. die den Wirkstoff der Formel I und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen des Wirkstoffs mit Streckmitteln, wie Lösungsmitteln, festen Trägerstoffen und gegebenenfalls oberflächenaktiven Verbindungen (Tensiden).

Als Lösungsmittel kommen in Frage: Aromatische Kohlenwasserstoffe, bevorzugt die Fraktionen $C_8$ bis $C_{12}$, wie Xylolgemische oder substituierte Naphthaline, Phthalsäureester, wie Dibutyl- oder Dioctylphthalat, aliphatische Kohlenwasserstoffe, wie Cyclohexan oder Paraffine, Alkohole und Glycole sowie deren Ether und Ester, wie Ethanol, Ethylenglycol, Ethylenglycolmonomethyl- oder -ethylether, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, und Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht-sorptive Trägermaterialien z.B. Calcit oder Sand in Frage. Darüber hinaus kann eine Vielzahl von vorgranulierten Materialien anorganischer oder organischer Natur, wie Dolomit oder zerkleinerte Pflanzenrückstände, verwendet werden.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen, als auch wasserlösliche synthetische oberflächenaktive Verbindungen sein.

Als Beispiele nichtionischer Tenside seien Nonylphenolpolyethoxyethanole, Ricinusölpolyglycolether, Polypropylen-/Polyethylen-oxidaddukte, Tributylphenoxypolyethylenethanol, Polyethylenglycol und Octylphenoxypolyethoxyethanol erwähnt.

Ferner kommen auch Fettsäureester von Polyoxyethylensorbitan, wie das Polyoxyethylensorbitantrioleat, in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige,

gegebenenfalls halogenierte Alkyl-, Benzyl- oder niedrige Hydroxyalkylreste aufweisen.

Weitere in der Formulierungstechnik gebräuchlichen Tenside sind dem Fachmann bekannt oder können der einschlägigen Fachliteratur entnommen werden.

Die agrochemischen Zubereitungen enthalten in der Regel 0,1 bis 99 Gewichtsprozent, insbesondere 0,1 bis 95 Gewichtsprozent, Wirkstoff der Formel I, 99,9 bis 1 Gewichtsprozent, insbesondere 99,8 bis 5 Gewichtsprozent, eines festen oder flüssigen Zusatzstoffes und 0 bis 25 Gewichtsprozent, insbesondere 0,1 bis 25 Gewichtsprozent, eines Tensids.

Während als Handelsware eher konzentrierte Mittel bevorzugt werden, verwendet der Endverbraucher in der Regel verdünnte Mittel.

Die Mittel können auch weitere Zusätze, wie Stabilisatoren, Entschäumer, Viskositätsregulatoren, Bindemittel oder Haftmittel, sowie Dünger oder andere Wirkstoffe zur Erzielung spezieller Effekte enthalten.

Die nachfolgenden Beispiele illustrieren die vorstehend beschriebene Erfindung, ohne dieselbe in ihrem Umfang in irgendeiner Weise einzuschränken. Temperaturen sind in Celsiusgraden angegeben.

Herstellungsbeispiele

H-1. Herstellung von 1(3)-Methylsulfonyl-5-chlor-6-(5-chlor-6-ethylpyrimid-4-yloxy)-benztriazol der Formel

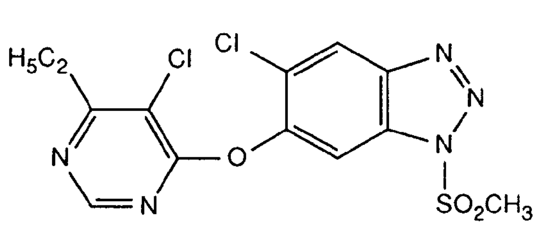

und

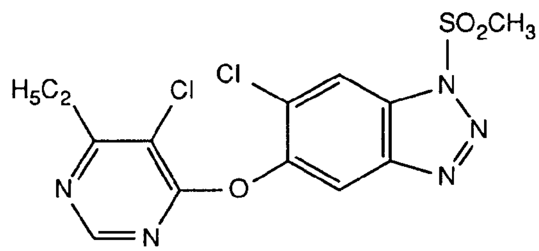

Herstellung der Zwischenprodukte

a) Herstellung von 5-Chlor-6-ethyl-3-(2-chlor-4-nitro-5-aminophenoxy)-pyrimidin.

Eine Lösung von 15,0 g 2-Chlor-4-amino-5-nitrophenol in 100 ml Dimethylsulfoxid wird mit 6,8 g Kaliumhydroxid versetzt und 30 Min. bei Raumtemperatur gerührt. Dazu gibt man eine Lösung von 15,5 g 4,5-Dichlor-6-ethylpyrimidin in 30 ml Dimethylsulfoxid und rührt während 90 Minuten bei 75°C. Die Reaktionslösung wird auf Wasser gegossen, der pH mit 5N Chlorwasserstoffsäure auf pH = 4 eingestellt und das Gemisch mit Essigsäureethylester extrahiert. Die organische Phase wird mit konz. NaCl-Lösung (Sole) gewaschen, über Magnesiumsulfat getrocknet und eingeengt. Chromatographie an Kieselgel mit Hexan/Diethylether (60/40) liefert 16,3 g des Zwischenprodukts a).

b) Herstellung von 5-Chlor-6-ethyl-3-(2-chlor-4,5-diaminophenoxy)-pyrimidin.

Eine Suspension von 15,3 g des unter a) erhaltenen Zwischenprodukts mit 3 g Raney-Nickel in 160 ml Tetrahydrofuran wird während 8 Stunden bei Raumtemperatur in einer Wasserstoffatmosphäre gerührt. Das Gemisch wird über Celite filtriert und eingeengt. Dies ergibt 13,6 g des Zwischenprodukts b), Smp. 160-165°C.

c) Herstellung von 5-Chlor-6-(5-chlor-6-ethylpyrimid-4-yloxy)-benztriazol.

Zu einer eisgekühlten Lösung von Natriumnitrit tropft man während 30 Minuten eine Lösung von 13,2 g des Zwischenprodukts b) in 50 ml Eisessig. Die resultierende Suspension wird bei Raumtemperatur 10 Stunden ausgerührt und filtriert. Der Filterrückstand wird mit Wasser gewaschen, in Essigsäureethylester gelöst, über Magnesiumsulfat getrocknet und eingeengt. Dies ergibt 12,7 g des Zwischenprodukts c), Smp. 149-151°C.

d) Herstellung des Isomerengemischs des Endprodukts Verb. Nr. 1.124

Eine Lösung von 5,8 g des Zwischenprodukts c) in 100 ml Aceton wird mit 1,4 g Kaliumhydroxid versetzt und 1 Stunde gerührt. Dazu gibt man 3,2 g Methylsulfonylchlorid in 30 ml Aceton und rührt die Mischung über Nacht. Nach Zugabe von 1 ml Triethylamin wird die Suspension noch 1 Stunde bei Raumtemperatur gerührt,

anschliessend durch Celite filtriert und das Filtrat eingeengt. Die Substanz wird in Dichlormethan gelöst, mit 5 g Kieselgel versetzt und fünf Minuten bei Raumtemperatur gerührt. Kieselgel wird abfiltriert und das Lösungsmittel verdampft. Umkristallisation aus Diethylether liefert 5,6 g der Stellungsisomeren, Smp. 137-140°C.

H-2. Herstellung von 1(3)-Methylsulfonyl-5-chlor-6-(3-chlor-5-trifluormethyl-pyrid-2-ylthio)-benztriazol der Formel

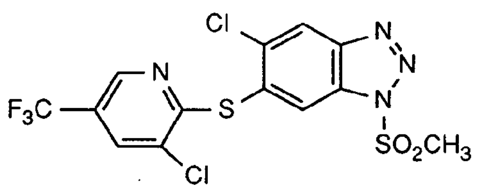

und

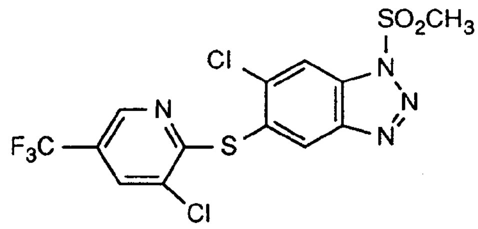

Herstellung der Zwischenprodukte

a) Herstellung von 2-Nitro-4-chlor-5-mercapto-anilin.

50,0 g 2-Nitro-4,5-dichlor-anilin werden in 250 ml Wasser und 250 ml Ethanol gelöst, mit 59,0 g $Na_2S{\cdot}8H_2O$ versetzt und 2,5 Stunden am Rückfluss gekocht. Nach Abkühlen auf Raumtemperatur wird die Reaktionslösung mit 1 Liter Wasser versetzt, filtriert und durch Zugabe von 25 ml Essigsäure sauer gestellt. Der gelbe Niederschlag wird abfiltriert, mit Wasser gewaschen und getrocknet.Dies ergibt 32,6 g der Verbindung a) (Smp. 183°C, Zersetzung).

b) Herstellung von 4-Chlor-5-mercapto-o-phenylendiamin.

255 g der Verbindung a) werden in 3 l Tetrahydrofuran gelöst und mit 255 g Platin(5%) auf Kohle versetzt und während 83 Stunden unter Wasserstoffatmosphäre hydriert. Der Katalysator wird abfiltriert und die Lösung eingeengt. Dies ergibt 163 g des Zwischenprodukts b), Smp. 158-160°C.

c) Herstellung von 1,2-Bis-(5-chlor-benztriazol-6-yl)-disulfid.

50,0 g der Verbindung b) werden in 350 ml Essigsäure gelöst und bei 0-5°C zu einer Lösung von 43,5 g Natriumnitrit in 1,75 l Wasser getropft. Die entstehende Suspension wird während 18 Stunden bei Raumtemperatur gerührt. Anschliessend wird filtriert, mit Wasser gewaschen und getrocknet. Dies liefert 48,5 g der Verbindung c) mit einem Smp. >250°C.

d) Herstellung von 5-Chlor-6-mercapto-benztriazol.

35,7 g der Verbindung c) werden in 250 ml Wasser suspendiert und mit 43,9 g $Na_2S_2O_4$ und 28,2 g $K_2CO_3$ versetzt und 1 Stunde am Rückfluss gekocht. Die entstandene braune Lösung wird filtriert und mit Chlorwasserstoffsäure angesäuert. Der Niederschlag wird abfiltriert und getrocknet. Dies ergibt 27,8 g der Verbindung d), Smp. 204-207°C.

e) Herstellung von 5-Chlor-6-(3-chlor-5-trifluormethyl-pyrid-2-ylthio)-benztriazol.

4,0 g der Verbindung d) werden in 30 ml n-Propanol suspendiert, mit 4,8 g 2,3-Dichlor-5-trifluormethylpyridin und 4,1 ml Triethylamin versetzt und 18 Stunden bei 70°C gerührt. Das Gemisch wird auf Chlorwasserstoffsäure gegossen, mit Essigsäureethylester extrahiert, über Natriumsulfat getrocknet, filtriert und eingeengt. Nach Chromatographie an Kieselgel mit Methylenchlorid/Ethanol 9:1 als Laufmittel erhält man 4,0 g der Verbindung e), Smp. 222-223°C.

f) Herstellung des Isomerengemischs des Endprodukts Verb. Nr. 2.161

3,5 g des Zwischenproduktes e) werden in 35 ml Aceton gelöst und mit 0,7 g KOH versetzt. Nach 2 Stunden Rühren bei Raumtemperatur gibt man 1,1 ml Methylsulfonylchlorid zu und rührt über Nacht. Danach wird die

Suspension abfiltriert und die Lösung eingeengt. Die Substanz wird in Dichlormethan gelöst, mit 5 g Kieselgel versetzt und 20 Minuten bei Raumtemperatur gerührt. Das Kieselgel wird abfiltriert und die Lösung eingeengt. Digerieren mit Diethylether und Trocknen der Kristalle liefert 2,7 g der Stellungsisomeren, Smp. 167-168°C.

H-3. Herstellung von 1-Methylsulfonyl-4-brom-6-(6-methoxy-pyridazin-3-yloxy)-benztriazol (Verb.Nr. 3.178)

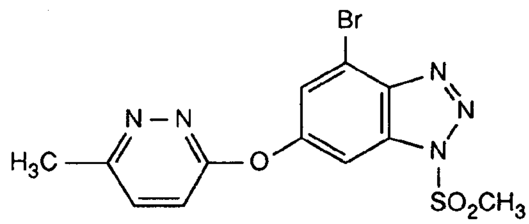

0,45 g (1,40 mMol) 6-(4-Methoxy-2,3-pyridazinyloxy)-4-brom-benztriazol werden in 10 ml Aceton suspendiert und bei Raumtemperatur nacheinander mit 0,58 g (4,19 mMol) wasserfreiem Kaliumcarbonat und 0,32 g (2,8 mMol) Methylsulfonylchlorid versetzt. Die Suspension wird 10 Minuten bei Raumtemperatur nachgerührt. Zur Aufarbeitung wird das Reaktionsgemisch im Eisbad gekühlt und mit 10-20 ml Eiswasser versetzt. Die dabei anfallenden Kristalle werden abfiltriert, nacheinander mit Wasser und Diethylether gewaschen und am Hochvakuum bei 40°C getrocknet. Man erhält 0,45 g (80% d.Th.) Produkt in Form beigefarbener Kristalle mit einem Schmelzpunkt unter Zersetzung von 187-188°C (unkorr.).

Auf diese Art oder nach Art einer der weiter oben angegebenen Verfahrensvarianten lassen sich folgende Verbindungen der Formel I herstellen.

Tabelle 1

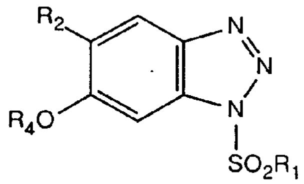

Isomerengemisch

| Verb. Nr. | $R_1$ | $R_2$ | $R_4$ | Smp. |
|---|---|---|---|---|
| 1.1 | $CH_3$ | H | 2-Pyridyl | |
| 1.2 | Et | Cl | 2-Pyridyl | |
| 1.3 | i-Pr | Br | 2-Pyridyl | |
| 1.4 | $N(CH_3)_2$ | F | 2-Pyridyl | |
| 1.5 | $CH_3$ | $CH_3$ | 3-Pyridyl | |
| 1.6 | $CH_3$ | Cl | 4-Pyridyl | |
| 1.7 | $CH_3$ | $CH_3O$ | 3-Cl-2-Pyridyl | |
| 1.8 | i-Pr | $CF_3$ | 3-Cl-2-Pyridyl | |
| 1.9 | $CF_3$ | $OCF_3$ | 4-Cl-2-Pyridyl | |
| 1.10 | $CH_3$ | Cl | 4-Cl-2-Pyridyl | |
| 1.11 | $N(CH_3)_2$ | H | 5-Cl-2-Pyridyl | |
| 1.12 | $CH_3$ | Br | 5-Cl-2-Pyridyl | |
| 1.13 | $CH_3$ | $C_2H_5$ | 6-Cl-2-Pyridyl | |
| 1.14 | Bu | $nC_4H_9$ | 4-Br-2-Pyridyl | |
| 1.15 | $CH_3$ | $N(CH_3)_2$ | 4-Br-2-Pyridyl | |
| 1.16 | $CH_3$ | Cl | 3-F-2-Pyridyl | |
| 1.17 | $CH_3$ | Br | $3-CF_3-2$-Pyridyl | |
| 1.18 | $CH_3$ | $OnC_4H_9$ | $4-CF_3-2$-Pyridyl | |
| 1.19 | $N(CH_3)_2$ | H | $5-CF_3-2$-Pyridyl | 58-63°C |
| 1.20 | $CH_3$ | H | $5-CF_3-2$-Pyridyl | 127-130°C |
| 1.21 | $CH_3$ | H | $6-CF_3-2$-Pyridyl | 105-109°C |
| 1.22 | $CH_3$ | $OCF_2H$ | $3-C_6F_{13}-2$-Pyridyl | |
| 1.23 | i-Pr | $OC_2F_5$ | $3-CH_3-2$-Pyridyl | |
| 1.24 | $CH_3$ | Cl | $5-CF_3-2$-Pyridyl | 131-144°C |

| Verb. Nr. | $R_1$ | $R_2$ | $R_4$ | Smp. |
|---|---|---|---|---|
| 1.25 | $nC_4H_9$ | $nC_4F_9$ | 4-$CH_3$-2-Pyridyl | |
| 1.26 | $CH_3$ | Cl | 4-$CH_3$-2-Pyridyl | |
| 1.27 | $CH_3$ | Br | 5-$CH_3$-2-Pyridyl | |
| 1.28 | $CH_3$ | H | 6-$CH_3$-2-Pyridyl | |
| 1.29 | $CH_3$ | $OnC_4H_9$ | 3-i-Pr-2-Pyridyl | |
| 1.30 | $CH_3$ | $N(nC_4H_9)_2$ | 4-n-Hex-2-Pyridyl | |
| 1.31 | $CH_3$ | F | 5-Et-2-Pyridyl | |
| 1.32 | $CH_3$ | H | 3-$OCH_2$-2-Pyridyl | |
| 1.33 | $CH_3$ | Br | 4-$OCH_3$-2-Pyridyl | |
| 1.34 | $CH_3$ | J | 5-OMe-2-Pyridyl | |
| 1.35 | $CH_3$ | $iC_3H_7$ | 6-OMe-2-Pyridyl | |
| 1.36 | $CH_3$ | $OC_2H_5$ | 4-$OEt_2$-2-Pyridyl | |
| 1.37 | $CH_3$ | $N(CH_3(C_2H_5)$ | 4-OiPr-2-Pyridyl | |
| 1.38 | $CH_3$ | $OCF_3$ | 3-OnHex-2-Pyridyl | |
| 1.39 | $N(CH_3)_2$ | $CF_3$ | 5-OtBu--2-Pyridyl | |
| 1.40 | $CH_3$ | Cl | 3-$OCF_3$-2-Pyridyl | |
| 1.41 | $CH_3$ | tert.$C_4H_9$ | 4-$OCF_3$-2-Pyridyl | |
| 1.42 | $CH_3$ | H | 5-$OCF_3$-2-Pyridyl | |
| 1.43 | $CH_3$ | H | 6-$OCF_3$-2-Pyridyl | |
| 1.44 | $CH_3$ | Otert.$C_4H_9$ | 3-$OCHF_2$-2-Pyridyl | |
| 1.45 | $CH_3$ | H | 4-$OCHF_2$-2-Pyridyl | |
| 1.46 | $CH_3$ | Cl | 5-$OCHF_2$-2-Pyridyl | |
| 1.47 | $CH_3$ | H | 6-$OCHF_2$-2-Pyridyl | |
| 1.48 | $CH_3$ | Br | 6-$OCH_2CF_3$-2-Pyridyl | |
| 1.49 | $CH_3$ | $C_2H_5$ | 6-$OC_6F_{13}$-2-Pyridyl | |
| 1.50 | $CH_3$ | $OiC_3H_7$ | 6-$NO_2$-2-Pyridyl | |
| 1.51 | $CH_3$ | $OnC_4H_9$ | 4-$NO_2$-2-Pyridyl | |
| 1.52 | Et | Cl | 3-$NO_2$-2-Pyridyl | |
| 1.53 | $N(CH_3)_2$ | H | 5-$NO_2$-2-Pyridyl | |
| 1.54 | $CH_3$ | Br | 6-SMe-2-Pyridyl | |
| 1.55 | $CH_3$ | $OCF_3$ | 6-S-Hexyl--2-Pyridyl | |
| 1.56 | $CH_3$ | H | 4-SMe-2-Pyridyl | |

| Verb. Nr. | $R_1$ | $R_2$ | $R_4$ | Smp. |
|---|---|---|---|---|
| 1.57 | $CH_3$ | $CH_2CF_3$ | 6-SiPr-2-Pyridyl | |
| 1.58 | $CH_3$ | Cl | 3-COOMe-2-Pyridyl | |
| 1.59 | i-Pr | H | 5-COOBu-2-Pyridyl | |
| 1.60 | $CH_3$ | $CF_3$ | 5-COOMe-2-Pyridyl | |
| 1.61 | $CH_3$ | Cl | 6-COOiPr-2-Pyridyl | |
| 1.62 | $CH_3$ | $OiC_3H_7$ | 5-CN-2-Pyridyl | |
| 1.63 | $CH_3$ | Cl | 6-CN-2-Pyridyl | |
| 1.64 | $CH_3$ | Br | 4-$N(CH_3)_2$-2-Pyridyl | |
| 1.65 | $CH_3$ | $OCF_3$ | 3-$N(Bu)_2$-2-Pyridyl | |
| 1.66 | $CH_3$ | H | 6-N(i-Pr)$_2$-2-Pyridyl | |
| 1.67 | $CH_3$ | $N(CH_3)_2$ | 4-$N(CH_3)$(Et)-2-Pyridyl | |
| 1.68 | $CH_3$ | Cl | 3,5-$(Cl)_2$-2-Pyridyl | 124-135°C |
| 1.69 | $CH_3$ | H | 3-Cl,5-F-2-Pyridyl | |
| 1.70 | $CH_3$ | $OCH_3$ | 3,4,5,6$(Cl)_4$-2-Pyridyl | |
| 1.71 | $CH_3$ | $CH_3$ | 3-$CF_3$, 5-Cl-2-Pyridyl | |
| 1.72 | $CH_3$ | H | 3-Cl, 5-$CF_3$-2-Pyridyl | 147-150°C |
| 1.73 | $CH_3$ | $OiC_3H_7$ | 6-Cl-3-$CF_3$-2-Pyridyl | |
| 1.74 | $CH_3$ | Cl | 3-Pyridazinyl | |
| 1.75 | $CH_3$ | H | 6-Cl-3-Pyridazinyl | |
| 1.76 | nBu | $OCF_3$ | 6-Br-4-$CF_3$-2-Pyridyl | |
| 1.77 | $CH_3$ | $N(CH_3)_2$ | 4-Cl, 3-$CH_3$-2-Pyridyl | |
| 1.78 | $CH_3$ | $OCF_3$ | 6-Cl-5-$CH_3$-2-Pyridyl | |
| 1.79 | $CH_3$ | $CH_3$ | 4-Br-5-$CH_3$-2-Pyridyl | |
| 1.80 | $CH_3$ | Cl | 6-$CH_3$-4-$CF_3$-2-Pyridyl | |
| 1.81 | $CH_3$ | H | 3-$CH_3$-4-Br-2-Pyridyl | |
| 1.82 | $CH_3$ | Cl | 4,6-$(CH_3)_2$-3-CN-2-Pyridyl | |
| 1.83 | $CH_3$ | Cl | 6-$(CH_3)$-4-(COOEt)-3-CN-2-Pyridyl | |
| 1.84 | $CH_3$ | H | 2-Br-3-Pyridyl | |
| 1.85 | $CH_3$ | Br | 2-Cl-3-Pyridyl | |
| 1.86 | $CH_3$ | $nC_4H_9$ | 2-J,6-$CH_3$-3-Pyridyl | |
| 1.87 | $CH_3$ | $OCH_3$ | 5-Cl-3-Pyridyl | |
| 1.88 | Et | Cl | 2,6$(Br_2)$-3-Pyridyl | |

| Verb. Nr. | $R_1$ | $R_2$ | $R_4$ | Smp. |
|---|---|---|---|---|
| 1.89 | $CH_3$ | H | $6(CH_3)$-3-Pyridyl | |
| 1.90 | $CH_3$ | Otert.$C_4H_9$ | 2-$NO_2$-3-Pyridyl | |
| 1.91 | $CH_3$ | i$C_3H_7$ | 2-$NO_2$-6-$CH_3$-3-Pyridyl | |
| 1.92 | $CH_3$ | $N(CH_3)_2$ | 2-[$N(CH_3)_2$]-3-Pyridyl | |
| 1.93 | $CH_3$ | n$C_4F_9$ | 2-COOMe-3-Pyridyl | |
| 1.94 | $CH_3$ | Cl | 5-$CF_3$-3-Pyridyl | |
| 1.95 | $CH_3$ | H | 2,3,5,6-($Cl_4$)-4-Pyridyl | |
| 1.96 | $CH_3$ | Cl | 3,5-$Cl_2$-4-Pyridyl | |
| 1.97 | $CH_3$ | $CH_3$ | 2-$CH_3$-4-Pyridyl | |
| 1.98 | $CH_3$ | Br | 2-COOMe-4-Pyridyl | |
| 1.99 | Et | sek.$C_4H_9$ | 2-Br-4-Pyridyl | |
| 1.100 | $N(CH_3)_2$ | F | 2-Cl-4-Pyridyl | |
| 1.101 | $CH_3$ | H | 2-$CF_3$-4-Pyridyl | |
| 1.102 | $CH_3$ | H | 2-Pyrimidyl | |
| 1.103 | $CH_3$ | $CF_3$ | 4-Pyrimidyl | |
| 1.104 | $CH_3$ | $OCH_3$ | 5-Pyrimidyl | |
| 1.105 | $CH_3$ | Br | 4-$CH_3$-2-Pyrimidyl | |
| 1.106 | $CH_3$ | Cl | 4,6-$(CH_3)_2$-2-Pyrimidyl | 63-67°C |
| 1.107 | $N(CH_3)_2$ | H | 4,6-$(CH_3)_2$-2-Pyrimidyl | |
| 1.108 | $CH_3$ | Oi$C_3H_7$ | 4-Cl-2-Pyrimidyl | |
| 1.109 | $CH_3$ | Cl | 4,6$(Cl)_2$-2-Pyrimidyl | |
| 1.110 | $CH_3$ | H | 4,5,6$(Cl)_3$-2-Pyrimidyl | |
| 1.111 | $CH_3$ | $CF_3$ | 4-Cl-6-$CH_3$-2-Pyrimidyl | |
| 1.112 | $CH_3$ | $OCF_3$ | 4-$CF_3$-5,6-$F_2$-2-Pyrimidyl | |
| 1.113 | Et | Cl | 4-$N(CH_3)_2$-5-$NO_2$-6-$CH_3$-2-Pyrimidyl | |
| 1.114 | $CH_3$ | $OCF_2$-$CF_2H$ | 4-$(CH_3)$-6$(OCH_3)$-2-Pyrimidyl | |
| 1.115 | $CH_3$ | Cl | 4,6-$F_2$-2-Pyrimidyl | |
| 1.116 | $CH_3$ | Br | 4-$(CF_3)$-5-COOMe-2-Pyrimidyl | |
| 1.117 | Bu | $N(CH_3)_2$ | 4-$N(Bu)_2$-5-F-2-Pyrimidyl | |
| 1.118 | $CH_3$ | H | 4-$OC_4F_9$-2-Pyrimidyl | |
| 1.119 | $CH_3$ | Cl | 4-(Cl)-5-$(CH_3)$-2-Pyrimidyl | 175-177°C |

| Verb. Nr. | $R_1$ | $R_2$ | $R_4$ | Smp. |
|---|---|---|---|---|
| 1.120 | $CH_3$ | H | 4,6-$(OMe)_2$-2-Pyrimidyl | |
| 1.121 | $CH_3$ | H | 2,6-$Cl_2$-1-Pyrimidyl | |
| 1.122 | $CH_3$ | Cl | 2-Cl,6-$CH_3$-4-Pyrimidyl | |
| 1.123 | i-Pr | Br | 2-Cl-5-$CH_3$-4-Pyrimidyl | |
| 1.124 | $CH_3$ | Cl | 5-Cl-6-Et-4-Pyrimidyl | 137-140°C |
| 1.125 | $CH_3$ | Cl | 2,6-$(MeO)_2$-4-Pyrimidyl | 159-160°C |
| 1.126 | $CH_3$ | Cl | 2-Cl,5-F-4-Pyrimidyl | |
| 1.127 | $CH_3$ | $OCF_3$ | 2,5-$F_2$-6-$CF_3$-4-Pyrimidyl | |
| 1.128 | $CH_3$ | $iC_3H_7$ | 2-Cl-6-$N(CH_3)_2$-4-Pyrimidyl | |
| 1.129 | $CH_3$ | F | 2,6-$F_2$-4-Pyrimidyl | |
| 1.130 | $CH_3$ | Cl | 2-SMe-6-Me-4-Pyrimidyl | |
| 1.131 | $CH_3$ | H | 2-S-Pr-4-Pyrimidyl | |
| 1.132 | $CH_3$ | Cl | 2-Cl-4-Pyrimidyl | |
| 1.133 | $CH_3$ | $OCH_3$ | 2-SMe-6-Cl-4-Pyrimidyl | |
| 1.134 | Bu | $OnC_3H_7$ | 2-$CH_3$,4-SMe,5-CN-4-Pyrimidyl | |
| 1.135 | $CH_3$ | H | 2-$CH_2$-5-Pyrimidyl | |
| 1.136 | $CH_3$ | Cl | 4-Cl-5-Pyrimidyl | |
| 1.137 | $CH_3$ | $CH_3$ | 2-SBu-5-Pyrimidyl | |
| 1.138 | $CH_3$ | $OCF_3$ | 2-CN-4-$NO_2$-5-Pyrimidyl | |
| 1.139 | $CH_3$ | $N(nC_4H_9)_2$ | 2-MeO-5-Pyrimidyl | |
| 1.140 | $N(CH_3)_2$ | H | 2-COOMe-5-Pyrimidyl | |
| 1.141 | $CH_3$ | Cl | 4-$N(Bu)_2$-5-Pyrimidyl | |
| 1.142 | $CH_3$ | Cl | 2,4-$Cl_2$-5-Pyrimidyl | |
| 1.143 | $CH_3$ | $CF_3$ | 2,4-$(CH_3)_2$-5-Pyrimidyl | |
| 1.144 | $CH_3$ | Cl | 2-Pyrazinyl | 195-196°C |
| 1.145 | $CH_3$ | Cl | 3-Chloro-2-Pyrazinyl | 149-152°C |
| 1.146 | $CH_3$ | Br | 6-Chloro-2-Pyrazinyl | |
| 1.147 | $CH_3$ | Cl | 3,6-$Me_2$-2-Pyrazinyl | |
| 1.148 | $CH_3$ | $CF_3$ | 3-Cl-5-$N(Me)_2$-6-COOMe-2-Pyrazinyl | |
| 1.149 | $N(CH_3)_2$ | $CH_3$ | 3,5-$(NBu_2)_2$-6-CN-2-Pyrazinyl | |
| 1.150 | Bu | $OCH_3$ | 3,5,6-$(CH_3)_3$-2-Pyrazinyl | |
| 1.151 | $CH_3$ | $OCF_2H$ | 3-Et-2-Pyrazinyl | |

| Verb. Nr. | $R_1$ | $R_2$ | $R_4$ | Smp. |
|---|---|---|---|---|
| 1.152 | $CH_3$ | Cl | 5-OMe-2-Pyrazinyl | |
| 1.153 | $CH_3$ | Br | 6-Br-2-Pyrazinyl | |
| 1.154 | $CH_3$ | H | 5-OMe-2-Pyrazinyl | |
| 1.155 | $CH_3$ | Cl | 3-$NO_2$-2-Pyrazinyl | |
| 1.156 | $CH_3$ | H | 3,5$(Cl)_2$-2-Pyridyl | 126-128°C |
| 1.157 | $N(CH_3)_2$ | H | 3,5$(Cl)_2$-2-Pyridyl | $n_D^{20}$= 1,5800 |
| 1.158 | $CH_3$ | H | 3-F,5Cl-2-Pyridyl | 109-112°C |
| 1.159 | $N(CH_3)_2$ | H | 3-F,5Cl-2-Pyridyl | 114-115°C |
| 1.160 | $CH_3$ | F | 3-Cl,5$(CF_3)$-2-Pyridyl | |
| 1.161 | $CH_3$ | Cl | 3-Cl,5$(CF_3)$-2-Pyridyl | 135-137°C |
| 1.162 | $CH_3$ | Br | 3-Cl,5$(CF_3)$-2-Pyridyl | 139-144°C |
| 1.163 | $CH_3$ | Cl | 4,6$(OCH_3)_2$-2-Pyrimidyl | |
| 1.164 | $CH_3$ | Br | 4,6$(OCH_3)_2$-2-Pyrimidyl | |
| 1.165 | $CH_3$ | Cl | 5-Cl,6-$OCH_3$-2-Pyrimidyl | 185-190°C |
| 1.166 | $N(CH_3)_2$ | Cl | 3-F,5-Cl-2-Pyridyl | 149-153°C |
| 1.167 | $CH_3$ | H | 5-Cl-6-n-propyl-4-Pyrimidyl | 120-126°C |
| 1.168 | $N(CH_3)_2$ | H | 5-Cl-6-n-propyl-4-Pyrimidyl | Harz |
| 1.169 | $N(CH_3)_2$ | Cl | 5-Cl-6-$CH_3$-4-Pyrimidyl | 110-112°C |
| 1.170 | $CH_3$ | Cl | 5-Cl-6-$CH_3$-4-Pyrimidyl | 146-150°C |
| 1.171 | $N(CH_3)_2$ | Cl | 5-$CH_3$-6-Cl-2-Pyrimidyl | 128-134°C |
| 1.172 | $CH_3$ | Cl | 5-$CH_3$-6-Cl-2-Pyrimidyl | 175-177°C |
| 1.173 | $N(CH_3)_2$ | H | 5-Cl-6-$CH_3$-4-Pyrimidyl | Harz |
| 1.174 | $CH_3$ | Cl | 4-$CH_3$-6-cycl.propyl-2-Pyrimidyl | 162-166°C |
| 1.175 | $N(CH_3)_2$ | Cl | 2-iPropyl-5-Cl-6-$CH_3$-2-Pyrimidyl | Harz |
| 1.176 | $CH_3$ | Cl | 6-$CF_3$-4-Pyrimidyl | 120-124°C |
| 1.177 | $CH_3$ | Cl | 2-iPropyl-5-Cl-6-$CH_3$-4-Pyrimidyl | 147-150°C |

| Verb. Nr. | $R_1$ | $R_2$ | $R_4$ | Smp. |
|---|---|---|---|---|
| 1.178 | $N(CH_3)_2$ | Cl | 6-$CF_3$-4-Pyrimidyl | 141-145°C |
| 1.179 | $CH_3$ | H | 5-Cl-6-$CH_3$-4-Pyrimidyl | 138-142°C |
| 1.180 | $CH_3$ | $CH_3$ | 3-Cl-5-$CF_3$-Pyridyl | 144-146°C |
| 1.181 | $CH_3$ | $OCH_3$ | 3-Cl-5-$CF_3$-Pyridyl | 130-135°C |
| 1.182 | $CH_3$ | H | 5-$CF_3$-6-Cl-Pyridyl | 128-135°C |
| 1.183 | $N(CH_3)_2$ | H | 5-$CF_3$-6-Cl-Pyridyl | 121-128°C |
| 1.184 | $N(CH_3)_2$ | $CF_3$ | 3-Cl-5-$CF_3$-Pyridyl | 153-156°C |
| 1.185 | $CH_3$ | Cl | 5-Cl-2-Pyridyl | 98-102°C |
| 1.186 | $N(CH_3)_2$ | H | 4-$CF_3$-2-Pyridyl | Oel |
| 1.187 | $N(CH_3)_2$ | H | 3-Cl-2-Pyrazinyl | 97-99°C |
| 1.188 | $N(CH_3)_2$ | Cl | 3-$CF_3$-5-Cl-2-Pyridyl | 120-129°C |
| 1.189 | $N(CH_3)_2$ | H | 3-$CF_3$-5-Cl-2-Pyridyl | fest |
| 1.190 | $CH_3$ | Cl | 3-$CF_3$-5-Cl-2-Pyridyl | 154-155°C |
| 1.191 | $N(CH_3)_2$ | H | 4-$CF_3$-6-$CH_3$-2-Pyridyl | Harz |
| 1.192 | $N(CH_3)_2$ | H | 5-Cl-6-Ethyl-4-Pyrimidyl | 104-105°C |
| 1.193 | $CH_3$ | H | 3-Cl-2-Pyrazin | 141-142°C |
| 1.194 | $N(CH_3)_2$ | H | 6-$CF_3$-2-Pyridyl | 133-135°C |
| 1.195 | $CH_3$ | H | 4-$CF_3$-2-Pyridyl | 80-82°C |
| 1.196 | $N(CH_3)_2$ | H | 3-$CF_3$-2-Pyridyl | 119-121°C |
| 1.197 | $CH_3$ | H | 6-Cl-2-Pyridyl | 98-100°C |
| 1.198 | $CH_3$ | H | 5-Cl-6-Ethyl-4-Pyrimidyl | 144-145°C |
| 1.199 | $CH_3$ | H | 4-$CF_3$-6-$CH_3$-2-Pyridyl | 88.5-100°C |
| 1.200 | $CH_3$ | H | 3-$CF_3$-2-Pyridyl | 182-185°C |
| 1.201 | $N(CH_3)_2$ | Br | 3-Cl-5-$CF_3$-2-Pyridyl | 109-111°C |
| 1.202 | $CH_3$ | H | 3-$CF_3$-5-Cl-2-Pyridyl | 101-102°C |
| 1.203 | $CH_3$ | Cl | 5-Cl-6-$CH_3$-2-$CH_3$-4-Pyrimidyl | |
| 1.204 | $CH_3$ | Cl | 4-$CH_3$-5-Cl-6-$CH_3$-2-Pyrimidyl | |
| 1.205 | $CH_3$ | Cl | 6-$OCH_3$-2-Pyrimidyl | |
| 1.206 | $CH_3$ | Cl | 4-$CH_3$-5-S-Ethyl-6-$CH_3$-2-Pyrimidyl | 125-126°C |
| 1.207 | $CH_3$ | H | 6-$CF_3$-2-Pyridyl | 124-125°C |
| 1.208 | $CH_3$ | Cl | 5-$CF_3$-2-Pyridyl | 163-164°C |

| Verb. Nr. | $R_1$ | $R_2$ | $R_4$ | Smp. |
|---|---|---|---|---|
| 1.209 | $CH_3$ | Cl | 3-Cl-5-$CF_3$-2-Pyridyl | 153-155°C |
| 1.210 | $CH_3$ | Br | 3-Cl-5-$CF_3$-2-Pyridyl | 177-178°C |
| 1.211 | $CH_3$ | Cl | 2,6-$(OCH_3)_2$-4-Pyrimidyl | 159-160°C |
| 1.212 | $CH_3$ | Cl | 2,6-$(CH_3)_2$-4-Pyrimidyl | 63-67°C |
| 1.213 | $CH_3$ | H | 2,6-$(OCH_3)_2$-4-Pyrimidyl | |
| 1.214 | $CH_3$ | H | 6-Cl-2-Pyrazinyl | |
| 1.215 | $CH_3$ | H | 3-Ethyl-2-Pyrazinyl | |
| 1.216 | $CH_3$ | H | 6-$CH_3$-3-Pyridazinyl | |
| 1.217 | $CH_3$ | H | 6-$OCH_3$-3-Pyridazinyl | |
| 1.218 | $CH_3$ | H | 6-Cl-3-Pyridazinyl | |
| 1.219 | $CH_3$ | H | 4,5-$Cl_2$-3-Pyridazinyl | |
| 1.220 | $N(CH_3)_2$ | H | 3-COOEt-2-Pyridyl | 139-140°C |
| 1.221 | $N(CH_3)_2$ | Cl | 6-$OCH_3$-4-Pyrimidyl | 135-144°C |
| 1.222 | $N(CH_3)_2$ | Cl | 4-$CH_3$-5-S-Ethyl-6-$CH_3$-2-Pyrimidyl | 97-98°C |
| 1.223 | $N(CH_3)_2$ | Cl | 3,5-$Cl_2$-2-Pyridyl | 125-127°C |

Tabelle 2

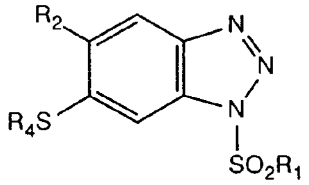

Isomerengemisch

| Verb. Nr. | $R_1$ | $R_2$ | $R_4$ | Smp. |
|---|---|---|---|---|
| 2.1 | $CH_3$ | H | 2-Pyridyl | 116-118°C |
| 2.2 | Et | Cl | 2-Pyridyl | |
| 2.3 | n-Pr | Br | 2-Pyridyl | |
| 2.4 | $N(CH_3)_2$ | F | 3-Pyridyl | |
| 2.5 | $CH_3$ | $CH_3$ | 3-Pyridyl | |
| 2.6 | $CH_3$ | Cl | 4-Pyridyl | 173-176°C |
| 2.7 | $CH_3$ | $CH_3O$ | 3-Cl-2-Pyridyl | |
| 2.8 | $CH_3$ | $CF_3$ | 3-Cl-2-Pyridyl | |
| 2.9 | $CF_3$ | $OCF_3$ | 4-Cl-2-Pyridyl | |
| 2.10 | $CH_3$ | Cl | 4-Cl-2-Pyridyl | |
| 2.11 | $N(CH_3)_2$ | H | 5-Cl-2-Pyridyl | |
| 2.12 | $CH_3$ | F | 5-Cl-2-Pyridyl | |
| 2.13 | $CH_3$ | $C_2H_5$ | 6-Cl-2-Pyridyl | |
| 2.14 | $CH_3$ | $nC_4H_9$ | 4-Br-2-Pyridyl | |
| 2.15 | $CH_3$ | $N(CH_3)_2$ | 4-Br-2-Pyridyl | |
| 2.16 | $CH_3$ | Cl | 3-F-2-Pyridyl | |
| 2.17 | $N(Et)_2$ | Br | $3-CF_3$-2-Pyridyl | |
| 2.18 | $CH_3$ | $OnC_4H_9$ | $4-CF_3$-2-Pyridyl | |
| 2.19 | $N(CH_3)_2$ | F | $5-C_6F_{13}$-2-Pyridyl | |
| 2.20 | $CH_3$ | Cl | $5-CF_3$-2-Pyridyl | |
| 2.21 | $CH_3$ | H | $6-CF_3$-2-Pyridyl | |
| 2.22 | $CH_3$ | $OCF_2H$ | $3-CF_3$-2-Pyridyl | |
| 2.23 | i-Pr | Cl | $3-CH_3$-2-Pyridyl | |
| 2.24 | $CH_3$ | H | $3-CH_3$-2-Pyridyl | |

| Verb. Nr. | $R_1$ | $R_2$ | $R_4$ | Smp. |
|---|---|---|---|---|
| 2.25 | $nC_4H_9$ | $nC_4F_9$ | 4-$CH_3$-2-Pyridyl | |
| 2.26 | $CH_3$ | Cl | 4-$CH_3$-2-Pyridyl | |
| 2.27 | $CH_3$ | Br | 5-$CH_3$-2-Pyridyl | |
| 2.28 | $CH_3$ | H | 6-$CH_3$-2-Pyridyl | |
| 2.29 | $CH_3$ | $OC_2F_5$ | 3-i-Pr-2-Pyridyl | |
| 2.30 | $CH_3$ | $N(nC_4H_9)_2$ | 4-n-Hex-2-Pyridyl | |
| 2.31 | $CH_3$ | F | 5-Et-2-Pyridyl | |
| 2.32 | $CH_3$ | H | 3-$OCH_2$-2-Pyridyl | |
| 2.33 | $CH_3$ | Br | 4-$OCH_3$-2-Pyridyl | |
| 2.34 | $CH_3$ | Cl | 5-OMe-2-Pyridyl | |
| 2.35 | $CH_3$ | $iC_3H_7$ | 6-OMe-2-Pyridyl | |
| 2.36 | $CH_3$ | $OC_2H_5$ | 4-$OEt_2$-2-Pyridyl | |
| 2.37 | $CH_3$ | $N(CH_3(C_2H_5)$ | 4-OiPr-2-Pyridyl | |
| 2.38 | $CH_3$ | $OCF_3$ | 3-OnHex-2-Pyridyl | |
| 2.39 | $N(CH_3)_2$ | $CF_3$ | 5-OtBu--2-Pyridyl | |
| 2.40 | $CH_3$ | Cl | 3-$OCF_3$-2-Pyridyl | |
| 2.41 | $CH_3$ | tert.$C_4H_9$ | 4-$OCF_3$-2-Pyridyl | |
| 2.42 | $CH_3$ | H | 5-$OCF_3$-2-Pyridyl | |
| 2.43 | $CH_3$ | H | 6-$OCF_3$-2-Pyridyl | |
| 2.44 | $CH_3$ | Otert.$C_4H_9$ | 3-$OCHF_2$-2-Pyridyl | |
| 2.45 | $CH_3$ | H | 4-$OCHF_2$-2-Pyridyl | |
| 2.46 | $CH_3$ | Cl | 5-$OCHF_2$-2-Pyridyl | |
| 2.47 | $CH_3$ | H | 6-$OCHF_2$-2-Pyridyl | |
| 2.48 | $CH_3$ | I | 6-$OCH_2CF_3$-2-Pyridyl | |
| 2.49 | $CH_3$ | $C_2H_5$ | 6-$OC_6F_{13}$-2-Pyridyl | |
| 2.50 | $CH_3$ | $OiC_3H_7$ | 6-$NO_2$-2-Pyridyl | |
| 2.51 | $CH_3$ | $OnC_4H_9$ | 4-$NO_2$-2-Pyridyl | |
| 2.52 | Et | Cl | 3-$NO_2$-2-Pyridyl | |
| 2.53 | $N(CH_3)_2$ | H | 5-$NO_2$-2-Pyridyl | |
| 2.54 | $CH_3$ | Br | 6-SMe-2-Pyridyl | |
| 2.55 | $CH_3$ | Cl | 6-S-Hexyl--2-Pyridyl | |
| 2.56 | $CH_3$ | H | 4-SMe-2-Pyridyl | |

| Verb. Nr. | $R_1$ | $R_2$ | $R_4$ | Smp. |
|---|---|---|---|---|
| 2.57 | $CH_3$ | $CH_2CF_3$ | 6-SiPr-2-Pyridyl | |
| 2.58 | $CH_3$ | Cl | 3-COOMe-2-Pyridyl | |
| 2.59 | i-Pr | H | 5-COOBu-2-Pyridyl | |
| 2.60 | $CH_3$ | $CF_3$ | 5-COOMe-2-Pyridyl | |
| 2.61 | $CH_3$ | Cl | 6-COOiPr-2-Pyridyl | |
| 2.62 | $CH_3$ | $OiC_3H_7$ | 5-CN-2-Pyridyl | |
| 2.63 | $CH_3$ | Cl | 6-CN-2-Pyridyl | |
| 2.64 | $CH_3$ | Br | 4-$N(CH_3)_2$-2-Pyridyl | |
| 2.65 | $CH_3$ | $OCF_3$ | 3-$N(Bu)_2$-2-Pyridyl | |
| 2.66 | $CH_3$ | H | 6-N(i-Pr)$_2$-2-Pyridyl | |
| 2.67 | $CH_3$ | $N(CH_3)_2$ | 4-$N(CH_3)$(Et)-2-Pyridyl | |
| 2.68 | $CH_3$ | H | 3,5-$(Cl)_2$-2-Pyridyl | |
| 2.69 | $CH_3$ | H | 3-Cl,5-F-2-Pyridyl | |
| 2.70 | $CH_3$ | $OCH_3$ | 3,4,5,6$(Cl)_4$-2-Pyridyl | |
| 2.71 | $CH_3$ | $CH_3$ | 3-$CF_3$, 5-Cl-2-Pyridyl | |
| 2.72 | $CH_3$ | H | 3-Cl, 5-$CF_3$-2-Pyridyl | |
| 2.73 | $CH_3$ | $nC_4H_9$ | 6-Cl-3-$CF_3$-2-Pyridyl | |
| 2.74 | $CH_3$ | $CH_3$ | 6-Cl-4-$CF_3$-2-Pyridyl | |
| 2.75 | $CH_3$ | H | 6-Cl-5-$CF_3$-2-Pyridyl | |
| 2.76 | nBu | $OCF_3$ | 6-Br-4-$CF_3$-2-Pyridyl | |
| 2.77 | $CH_3$ | $N(CH_3)_2$ | 4-Cl, 3-$CH_3$-2-Pyridyl | |
| 2.78 | $CH_3$ | $OCF_3$ | 6-Cl-5-$CH_3$-2-Pyridyl | |
| 2.79 | $CH_3$ | $CH_3$ | 4-Br-5-$CH_3$-2-Pyridyl | |
| 2.80 | $CH_3$ | Cl | 6-$CH_3$-4-$CF_3$-2-Pyridyl | |
| 2.81 | $CH_3$ | H | 3-$CH_3$-4-Br-2-Pyridyl | |
| 2.82 | $CH_3$ | Cl | 4,6-$(CH_3)_2$-3-CN-2-Pyridyl | 144-154°C |
| 2.83 | $CH_3$ | Cl | 4-$CH_3$-3-Pyridazinyl | |
| 2.84 | $CH_3$ | H | 2-Br-3-Pyridyl | |
| 2.85 | $CH_3$ | Br | 2-Cl-3-Pyridyl | |
| 2.86 | $CH_3$ | H | 2-I,6-$CH_3$-3-Pyridyl | |
| 2.87 | $CH_3$ | $OCH_3$ | 5-Cl-3-Pyridyl | |

| Verb. Nr. | $R_1$ | $R_2$ | $R_4$ | Smp. |
|---|---|---|---|---|
| 2.88 | Et | Cl | 2,6($Br_2$)-3-Pyridyl | |
| 2.89 | $CH_3$ | H | 6($CH_3$)-3-Pyridyl | |
| 2.90 | $CH_3$ | Otert.$C_4H_9$ | 2-$NO_2$-3-Pyridyl | |
| 2.91 | $CH_3$ | i$C_3H_7$ | 2-$NO_2$-6-$CH_3$-3-Pyridyl | |
| 2.92 | $CH_3$ | Cl | 2-[N($CH_3$)$_2$]-3-Pyridyl | |
| 2.93 | $CH_3$ | n$C_4F_9$ | 2-COOMe-3-Pyridyl | |
| 2.94 | $CH_3$ | Cl | 5-$CF_3$-3-Pyridyl | |
| 2.95 | $CH_3$ | H | 2,3,5,6-($Cl_4$)-4-Pyridyl | |
| 2.96 | $CH_3$ | Cl | 3,5-$Cl_2$-4-Pyridyl | |
| 2.97 | $CH_3$ | $CH_3$ | 2-$CH_3$-4-Pyridyl | |
| 2.98 | $CH_3$ | Br | 2-COOMe-4-Pyridyl | |
| 2.99 | $CH_3$ | Cl | 2-Pyridyl | 158-161°C |
| 2.100 | N($CH_3$)$_2$ | F | 2-Cl-4-Pyridyl | |
| 2.101 | $CH_3$ | H | 2-$CF_3$-4-Pyridyl | |
| 2.102 | $CH_3$ | H | 2-Pyrimidyl | |
| 2.103 | $CH_3$ | $CF_3$ | 4-Pyrimidyl | |
| 2.104 | $CH_3$ | $OCH_3$ | 5-Pyrimidyl | |
| 2.105 | $CH_3$ | Br | 4-$CH_3$-2-Pyrimidyl | |
| 2.106 | $CH_3$ | Cl | 4,6-($CH_3$)$_2$-2-Pyrimidyl | 165-168,5°C |
| 2.107 | N($CH_3$)$_2$ | H | 4,6-($CH_3$)$_2$-2-Pyrimidyl | |
| 2.108 | $CH_3$ | Oi$C_3H_7$ | 4-Cl-2-Pyrimidyl | |
| 2.109 | $CH_3$ | Cl | 4,6(Cl)$_2$-2-Pyrimidyl | |
| 2.110 | $CH_3$ | H | 4,5,6(Cl)$_3$-2-Pyrimidyl | |
| 2.111 | $CH_3$ | $CF_3$ | 4-Cl-6-$CH_3$-2-Pyrimidyl | |
| 2.112 | $CH_3$ | $OCF_3$ | 4-$CF_3$-5,6-$F_2$-2-Pyrimidyl | |
| 2.113 | Et | Cl | 4-N($CH_3$)$_2$-5-$NO_2$-6-$CH_3$-2-Pyrimidyl | |
| 2.114 | $CH_3$ | $OCF_2$-$CF_2H$ | 6($OCH_3$)-2-Pyrimidyl | |
| 2.115 | $CH_3$ | Cl | 4,6-$F_2$-2-Pyrimidyl | |
| 2.116 | $CH_3$ | Br | 4-($CF_3$)-5-COOMe-2-Pyrimidyl | |
| 2.117 | Bu | N($CH_3$)$_2$ | 4-N(Bu)$_2$-5-F-2-Pyrimidyl | |
| 2.118 | $CH_3$ | H | 4-$OC_4F_9$-2-Pyrimidyl | |
| 2.119 | $CH_3$ | Cl | 3-Pyridazinyl | |

| Verb. Nr. | $R_1$ | $R_2$ | $R_4$ | Smp. |
|---|---|---|---|---|
| 2.120 | $CH_3$ | H | 4,6-$(OMe)_2$-2-Pyrimidyl | |
| 2.121 | $CH_3$ | H | 2,6-$Cl_2$-4-Pyrimidyl | |
| 2.122 | $CH_3$ | Cl | 2-Cl,6-$CH_3$-4-Pyrimidyl | |
| 2.123 | i-Pr | Br | 2-Cl-5-$CH_3$-4-Pyrimidyl | |
| 2.124 | $CH_3$ | Cl | 5-Cl-6-Et-4-Pyrimidyl | 176-183°C |
| 2.125 | $CH_3$ | H | 2,6-$(MeO)_2$-4-Pyrimidyl | |
| 2.126 | $CH_3$ | Cl | 2-Cl,5-F-4-Pyrimidyl | |
| 2.127 | $CH_3$ | $OCF_3$ | 2,5-$F_2$-6-$CF_3$-4-Pyrimidyl | |
| 2.128 | $CH_3$ | Cl | 2-Cl-6-$N(CH_3)_2$-4-Pyrimidyl | |
| 2.129 | $CH_3$ | F | 2,6-$F_2$-4-Pyrimidyl | |
| 2.130 | $CH_3$ | Cl | 2-SMe-6-Me-4-Pyrimidyl | |
| 2.131 | $CH_3$ | H | 2-S-Pr-4-Pyrimidyl | |
| 2.132 | $CH_3$ | Cl | 2-Cl-4-Pyrimidyl | |
| 2.133 | $CH_3$ | $OCH_3$ | 2-SMe-6-Cl-4-Pyrimidyl | |
| 2.134 | Bu | $OnC_3H_7$ | 2-$CH_3$,4-SMe,5-CN-4-Pyrimidyl | |
| 2.135 | $CH_3$ | H | 2-$CH_2$-5-Pyrimidyl | |
| 2.136 | $CH_3$ | Cl | 4-Cl-5-Pyrimidyl | |
| 2.137 | $CH_3$ | $CH_3$ | 2-SBu-5-Pyrimidyl | |
| 2.138 | $CH_3$ | $OCF_3$ | 2-CN-4-$NO_2$-5-Pyrimidyl | |
| 2.139 | $CH_3$ | H | 2-MeO-5-Pyrimidyl | |
| 2.140 | $N(CH_3)_2$ | H | 2-COOMe-5-Pyrimidyl | |
| 2.141 | $CH_3$ | Cl | 4-$N(Bu)_2$-5-Pyrimidyl | |
| 2.142 | $CH_3$ | $N(nC_4H_9)_2$ | 2,4-$Cl_2$-5-Pyrimidyl | |
| 2.143 | $CH_3$ | $CF_3$ | 2,4-$(CH_3)_2$-5-Pyrimidyl | |
| 2.144 | $CH_3$ | H | 2-Pyrazinyl | |
| 2.145 | $CH_3$ | Cl | 3-Chloro-2-Pyrazinyl | 152-154°C |
| 2.146 | $CH_3$ | Br | 6-Chloro-2-Pyrazinyl | |
| 2.147 | $CH_3$ | Cl | 3,6-$Me_2$-2-Pyrazinyl | 131-133°C |
| 2.148 | $CH_3$ | Cl | 3-Cl-5-$N(Me)_2$-6-COOMe-2-Pyrazinyl | |
| 2.149 | $N(CH_3)_2$ | $CH_3$ | 3,5-$(NBu_2)_2$-6-CN-2-Pyrazinyl | |
| 2.150 | Bu | $OCH_3$ | 3,5,6-$(CH_3)_3$-2-Pyrazinyl | |
| 2.151 | $CH_3$ | H | 3-Et-2-Pyrazinyl | |

| Verb. Nr. | $R_1$ | $R_2$ | $R_4$ | Smp. |
|---|---|---|---|---|
| 2.152 | $CH_3$ | Cl | 5-OMe-2-Pyrazinyl | |
| 2.153 | $CH_3$ | Br | 6-Br-2-Pyrazinyl | |
| 2.154 | $CH_3$ | $OCF_2H$ | 5-OMe-2-Pyrazinyl | |
| 2.155 | $CH_3$ | Cl | 3-$NO_2$-2-Pyrazinyl | |
| 2.156 | $CH_3$ | Br | 3,5(Cl)$_2$-2-Pyridyl | |
| 2.157 | $CH_3$ | Cl | 3,5(Cl)$_2$-2-Pyridyl | 141-143°C |
| 2.158 | $CH_3$ | Cl | 3-F,5Cl-2-Pyridyl | |
| 2.159 | $CH_3$ | Br | 3-Cl,5F-2-Pyridyl | |
| 2.160 | $CH_3$ | F | 3-Cl,5($CF_3$)-2-Pyridyl | |
| 2.161 | $CH_3$ | Cl | 3-Cl,5($CF_3$)-2-Pyridyl | 167-168°C |
| 2.162 | $CH_3$ | Br | 3-Cl,5($CF_3$)-2-Pyridyl | |
| 2.163 | $CH_3$ | Cl | 4,6($OCH_3$)$_2$-2-Pyrimidyl | |
| 2.164 | $CH_3$ | Br | 4,6($OCH_3$)$_2$-2-Pyrimidyl | |
| 2.165 | $CH_3$ | Cl | 6-Cl-2-Pyrazinyl | 147-149°C |
| 2.166 | $CH_3$ | Cl | 6-$OCH_3$-3-Pyridazinyl | 176-179°C |
| 2.167 | $CH_3$ | Cl | 6-$CH_3$-3-Pyridazinyl | 174-177°C |
| 2.168 | $CH_3$ | Cl | 6-Cl-3-Pyridazinyl | 187-190°C |
| 2.169 | $CH_3$ | H | 4-$CH_3$-2-Pyrimidyl | 103-106°C |
| 2.170 | $CH_3$ | Br | 4,6-($CH_3$)$_2$-2-Pyrimidyl | 152-154°C |
| 2.171 | $CH_3$ | Cl | 5-Cl-6-(n-$C_3H_7$)-4-Pyrimidyl | 164-165°C |
| 2.172 | $CH_3$ | Cl | 5-Cl-6-$CH_3$-4-Pyrimidyl | 175-176°C |
| 2.173 | $CH_3$ | Cl | 2-$SCH_3$-5-$OCH_3$-4-Pyrimidyl | 165-170°C |
| 2.174 | $CH_3$ | Cl | 2,6-($OCH_3$)$_2$-4-Pyrimidyl | 179-181°C |
| 2.175 | $CH_3$ | Cl | 2,6-$Cl_2$-4-Pyrimidyl | 219-225°C |
| 2.176 | $CH_3$ | Cl | 2-Pyrimidyl | 130-134°C |
| 2.177 | $CH_3$ | H | 5-$CF_3$-2-Pyridyl | 107-109°C |

Tabelle 3

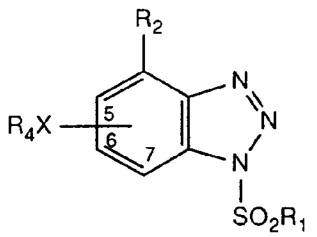

Einzelisomer

| Verb. Nr. | $R_1$ | $R_2$ | $R_4$ | X | Smp. |
|---|---|---|---|---|---|
| 3.1 | $CH_3$ | H | 2-Pyridyl | 5-S | |
| 3.2 | Et | Cl | 2-Pyridyl | 6-O | |
| 3.3 | i-Pr | F | 2-Pyridyl | 6-S | |
| 3.4 | $N(CH_3)_2$ | Br | 2-Pyridyl | 5-O | |
| 3.5 | $CH_3$ | $CH_3$ | 3-Pyridyl | 6-O | |
| 3.6 | $CH_3$ | Cl | 4-Pyridyl | 6-S | |
| 3.7 | $CH_3$ | $CH_3O$ | 3-Cl-2-Pyridyl | 5-S | |
| 3.8 | i-Pr | $CF_3$ | 3-Cl-2-Pyridyl | 6-O | |
| 3.9 | $CH_3$ | $OCF_3$ | 4-Cl-2-Pyridyl | 6-S | |
| 3.10 | $CH_3$ | Cl | 4-Cl-2-Pyridyl | 6-O | |
| 3.11 | $N(CH_3)_2$ | Br | 5-Cl-2-Pyridyl | 5-S | |
| 3.12 | $CH_3$ | J | 5-Cl-2-Pyridyl | 6-S | |
| 3.13 | $CH_3$ | $C_2H_5$ | 6-Cl-2-Pyridyl | 6-O | |
| 3.14 | Bu | Br | 4-Br-2-Pyridyl | 6-O | |
| 3.15 | $CH_3$ | Cl | 4-Br-2-Pyridyl | 5-S | |
| 3.16 | $CH_3$ | Cl | 3-F-2-Pyridyl | 6-S | |
| 3.17 | $CH_3$ | Br | 3-$CF_3$-2-Pyridyl | 5-O | |
| 3.18 | $CH_3$ | $OnC_4H_9$ | 4-$CF_3$-2-Pyridyl | 5-O | |
| 3.19 | $N(CH_3)_2$ | Br | 5-$CF_3$-2-Pyridyl | 6-O | |
| 3.20 | $CH_3$ | Cl | 5-$CF_3$-2-Pyridyl | 6-O | 157-159°C |
| 3.21 | $CH_3$ | Br | 6-$CF_3$-2-Pyridyl | 5-S | |
| 3.22 | $CH_3$ | $OCH_3$ | 3-$C_6F_{13}$-2-Pyridyl | 6-O | |
| 3.23 | $CH_3$ | $OC_2F_5$ | 3-$CH_3$-2-Pyridyl | 6-S | |

| Verb. Nr. | $R_1$ | $R_2$ | $R_4$ | X | Smp. |
|---|---|---|---|---|---|
| 3.24 | $CH_3$ | Cl | 3-$CH_3$-2-Pyridyl | 5-S | |
| 3.25 | $N(CH_3)_2$ | $nC_4F_9$ | 4-$CH_3$-2-Pyridyl | 5-S | |
| 3.26 | $CH_3$ | Cl | 4-$CH_3$-2-Pyridyl | 6-O | |
| 3.27 | $CH_3$ | Br | 5-$CH_3$-2-Pyridyl | 6-S | |
| 3.28 | $CH_3$ | Br | 6-$CH_3$-2-Pyridyl | 5-O | |
| 3.29 | $CH_3$ | $OnC_4H_9$ | 3-i-Pr-2-Pyridyl | 6-S | |
| 3.30 | $CH_3$ | $N(nC_4H_9)_2$ | 4-n-Hex-2-Pyridyl | 6-S | |
| 3.31 | $CH_3$ | F | 5-Et-2-Pyridyl | 5-S | |
| 3.32 | $CH_3$ | Br | 3-$OCH_2$-2-Pyridyl | 5-O | |
| 3.33 | $CH_3$ | Br | 4-$OCH_3$-2-Pyridyl | 6-S | |
| 3.34 | $CH_3$ | Cl | 5-OMe-2-Pyridyl | 6-O | |
| 3.35 | $CH_3$ | $iC_3H_7$ | 6-OMe-2-Pyridyl | 6-O | |
| 3.36 | $CH_3$ | $OC_2H_5$ | 4-$OEt_2$-2-Pyridyl | 6-O | |
| 3.37 | $CH_3$ | $N(CH_3(C_2H_5)$ | 4-OiPr-2-Pyridyl | 5-S | |
| 3.38 | $CH_3$ | Br | 3-OnHex-2-Pyridyl | 6-S | |
| 3.39 | $N(CH_3)_2$ | $CF_3$ | 5-OtBu--2-Pyridyl | 5-S | |
| 3.40 | $CH_3$ | Cl | 3-$OCF_3$-2-Pyridyl | 6-O | |
| 3.41 | $CH_3$ | tert.$C_4H_9$ | 4-$OCF_3$-2-Pyridyl | 6-S | |
| 3.42 | $CH_3$ | Br | 5-$OCF_3$-2-Pyridyl | 5-S | |
| 3.43 | $CH_3$ | Br | 6-$OCF_3$-2-Pyridyl | 5-O | |
| 3.44 | $CH_3$ | Otert.$C_4H_9$ | 3-$OCHF_2$-2-Pyridyl | 6-O | |
| 3.45 | $CH_3$ | Br | 4-$OCHF_2$-2-Pyridyl | 5-S | |
| 3.46 | $CH_3$ | Cl | 5-$OCHF_2$-2-Pyridyl | 6-S | |
| 3.47 | $CH_3$ | Br | 6-$OCHF_2$-2-Pyridyl | 5-O | |
| 3.48 | $N(CH_3)_2$ | Br | 6-$OCH_2CF_3$-2-Pyridyl | 6-O | |
| 3.49 | $CH_3$ | Br | 6-$OC_6F_{13}$-2-Pyridyl | 6-S | |
| 3.50 | $CH_3$ | $OiC_3H_7$ | 6-$NO_2$-2-Pyridyl | 5-S | |
| 3.51 | $CH_3$ | $OnC_4H_9$ | 4-$NO_2$-2-Pyridyl | 6-O | |
| 3.52 | Et | Cl | 3-$NO_2$-2-Pyridyl | 6-S | |
| 3.53 | $N(CH_3)_2$ | Br | 5-$NO_2$-2-Pyridyl | 5-S | |
| 3.54 | $CH_3$ | Br | 6-SMe-2-Pyridyl | 6-O | |
| 3.55 | $CH_3$ | $OCF_3$ | 6-S-Hexyl--2-Pyridyl | 5-S | |

| Verb. Nr. | $R_1$ | $R_2$ | $R_4$ | X | Smp. |
|---|---|---|---|---|---|
| 3.56 | $CH_3$ | $OCH_3$ | 4-SMe-2-Pyridyl | 5-O | |
| 3.57 | $CH_3$ | $CH_2CF_3$ | 5-Cl-2-Pyridyl | 6-O | |
| 3.58 | $CH_3$ | Cl | 3-COOMe-2-Pyridyl | 6-S | |
| 3.59 | i-Pr | $N(CH_3)_2$ | 5-COOBu-2-Pyridyl | 5-S | |
| 3.60 | $CH_3$ | $CF_3$ | 5-COOMe-2-Pyridyl | 6-O | |
| 3.61 | $CH_3$ | Cl | 6-COOiPr-2-Pyridyl | 5-O | |
| 3.62 | $CH_3$ | Br | 5-$CH_3$-2-Pyridyl | 6-S | |
| 3.63 | $CH_3$ | Cl | 6-CN-2-Pyridyl | 6-O | |
| 3.64 | $CH_3$ | Br | 4-$N(CH_3)_2$-2-Pyridyl | 5-S | |
| 3.65 | $CH_3$ | $OC_2H_5$ | 3-$N(Bu)_2$-2-Pyridyl | 6-S | |
| 3.66 | $CH_3$ | $CF_3$ | 6-N(i-Pr)$_2$-2-Pyridyl | 5-O | |
| 3.67 | $CH_3$ | Br | 4-$N(CH_3)$(Et)-2-Pyridyl | 6-O | |
| 3.68 | $CH_3$ | Br | 3,5-$(Cl)_2$-2-Pyridyl | 5-O | |
| 3.69 | $CH_3$ | Br | 3-Cl,5-F-2-Pyridyl | 5-S | |
| 3.70 | $CH_3$ | $OCH_3$ | 3,4,5,6$(Cl)_4$-2-Pyridyl | 6-O | |
| 3.71 | $CH_3$ | $CH_3$ | 3-$CF_3$, 5-Cl-2-Pyridyl | 6-O | |
| 3.72 | $CH_3$ | Br | 3-Cl, 5-$CF_3$-2-Pyridyl | 5-S | |
| 3.73 | $CH_3$ | $OCH_3$ | 6-Cl-3-$CF_3$-2-Pyridyl | 6-S | |
| 3.74 | $CH_3$ | $CH_3$ | 6-Cl-4-$CF_3$-2-Pyridyl | 5-S | |
| 3.75 | $CH_3$ | $OCH_3$ | 6-Cl-5-$CF_3$-2-Pyridyl | 5-O | |
| 3.76 | nBu | Br | 6-Br-4-$CF_3$-2-Pyridyl | 6-O | |
| 3.77 | $CH_3$ | $N(CH_3)_2$ | 4-Cl, 3-$CH_3$-2-Pyridyl | 6-S | |
| 3.78 | $CH_3$ | $OCF_3$ | 6-Cl-5-$CH_3$-2-Pyridyl | 5-S | |
| 3.79 | $CH_3$ | $CH_3$ | 4-Br-5-$CH_3$-2-Pyridyl | 6-O | |
| 3.80 | $CH_3$ | Cl | 6-$CH_3$-4-$CF_3$-2-Pyridyl | 5-O | |
| 3.81 | $N(CH_3)_2$ | $C_2H_5$ | 3-$CH_3$-4-Br-2-Pyridyl | 5-S | |
| 3.82 | $CH_3$ | Cl | 4,6-$(CH_3)_2$-3-CN-2-Pyridyl | 6-O | |
| 3.83 | $CH_3$ | Br | 3-Pyridazinyl | 6-S | |
| 3.84 | $CH_3$ | Br | 2-Br-3-Pyridyl | 5-S | |
| 3.85 | $CH_3$ | Br | 2-Cl-3-Pyridyl | 6-O | |
| 3.86 | $CH_3$ | Cl | 2-J,6-$CH_3$-3-Pyridyl | 6-S | |
| 3.87 | $CH_3$ | $OCH_3$ | 5-Cl-3-Pyridyl | 5-S | |

| Verb. Nr. | $R_1$ | $R_2$ | $R_4$ | X | Smp. |
|---|---|---|---|---|---|
| 3.88 | Et | Cl | 2,6($Br_2$)-3-Pyridyl | 6-O | |
| 3.89 | $CH_3$ | Br | 6($CH_3$)-3-Pyridyl | 5-O | |
| 3.90 | $CH_3$ | Br | 2-$NO_2$-3-Pyridyl | 6-O | |
| 3.91 | $CH_3$ | $iC_3H_7$ | 2-$NO_2$-6-$CH_3$-3-Pyridyl | 5-S | |
| 3.92 | $CH_3$ | Br | 2-[$N(CH_3)_2$]-3-Pyridyl | 5-O | |
| 3.93 | $CH_3$ | $nC_4F_9$ | 2-COOMe-3-Pyridyl | 6-S | |
| 3.94 | $N(CH_3)_2$ | Cl | 5-$CF_3$-3-Pyridyl | 5-S | |
| 3.95 | $CH_3$ | Br | 2,3,5,6-($Cl_4$)-4-Pyridyl | 5-O | |
| 3.96 | $CH_3$ | Cl | 3,5-$Cl_2$-4-Pyridyl | 6-S | |
| 3.97 | $CH_3$ | $CH_3$ | 2-$CH_3$-4-Pyridyl | 6-S | |
| 3.98 | $CH_3$ | Br | 2-COOMe-4-Pyridyl | 6-O | |
| 3.99 | Et | Br | 2-Br-4-Pyridyl | 5-O | |
| 3.100 | $N(CH_3)_2$ | F | 2-Cl-4-Pyridyl | 6-S | |
| 3.101 | $CH_3$ | Br | 2-$CF_3$-4-Pyridyl | 5-S | |
| 3.102 | $CH_3$ | $CH_3$ | 2-Pyrimidyl | 5-O | |
| 3.103 | $CH_3$ | $CF_3$ | 4-Pyrimidyl | 5-S | |
| 3.104 | $CH_3$ | $OCH_3$ | 5-Pyrimidyl | 6-O | |
| 3.105 | $CH_3$ | Br | 4-$CH_3$-2-Pyrimidyl | 6-S | |
| 3.106 | $CH_3$ | Cl | 4,6-$(CH_3)_2$-2-Pyrimidyl | 6-S | |
| 3.107 | $N(CH_3)_2$ | $OCF_3$ | 4,6-$(CH_3)_2$-2-Pyrimidyl | 5-S | |
| 3.108 | $CH_3$ | $OiC_3H_7$ | 4-Cl-2-Pyrimidyl | 6-O | |
| 3.109 | $CH_3$ | Cl | 4,6$(Cl)_2$-2-Pyrimidyl | 5-S | |
| 3.110 | $CH_3$ | $C_2H_5$ | 4,5,6$(Cl)_3$-2-Pyrimidyl | 5-O | |
| 3.111 | $CH_3$ | Br | 4-Cl-6-$CH_3$-2-Pyrimidyl | 6-O | |
| 3.112 | $CH_3$ | $OCF_3$ | 4-$CF_3$-5,6-$F_2$-2-Pyrimidyl | 5-S | |
| 3.113 | $CH_3$ | Cl | 4-$CH_3$-3-Pyridazinyl | 6-O | |
| 3.114 | $CH_3$ | Br | 4-($CH_3$)-6($OCH_3$)-2-Pyrimidyl | 6-O | |
| 3.115 | $CH_3$ | Cl | 4,6-$F_2$-2-Pyrimidyl | 6-O | |
| 3.116 | $CH_3$ | Br | 4-($CF_3$)-5-COOMe-2-Pyrimidyl | 6-O | |
| 3.117 | Bu | $N(CH_3)_2$ | 4-$N(Bu)_2$-5-F-2-Pyrimidyl | 5-S | |
| 3.118 | $CH_3$ | Br | 4-$OC_4F_9$-2-Pyrimidyl | 5-S | |
| 3.119 | $N(CH_3)_2$ | Cl | 4-(Cl)-5-($CH_3$)-2-Pyrimidyl | 6-S | |

| Verb. Nr. | $R_1$ | $R_2$ | $R_4$ | X | Smp. |
|---|---|---|---|---|---|
| 3.120 | $CH_3$ | Br | 4,6-$(OMe)_2$-2-Pyrimidyl | 5-O | |
| 3.121 | $CH_3$ | Cl | 2,6-$Cl_2$-1-Pyrimidyl | 5-O | |
| 3.122 | $CH_3$ | $OCF_2$-$CF_2H$ | 2-Cl,6-$CH_3$-4-Pyrimidyl | 6-O | |
| 3.123 | i-Pr | Br | 2-Cl-5-$CH_3$-4-Pyrimidyl | 6-S | |
| 3.124 | $CH_3$ | Cl | 5-Cl-6-Et-4-Pyrimidyl | 6-S | |
| 3.125 | $CH_3$ | Br | 2,6-$(MeO)_2$-4-Pyrimidyl | 5-S | |
| 3.126 | Bu | Cl | 2-Cl,5-F-4-Pyrimidyl | 6-O | |
| 3.127 | $CH_3$ | $OCF_3$ | 2,5-$F_2$-6-$CF_3$-4-Pyrimidyl | 6-O | |
| 3.128 | $CH_3$ | Cl | 2-Cl-6-$N(CH_3)_2$-4-Pyrimidyl | 5-S | |
| 3.129 | $CH_3$ | F | 2,6-$F_2$-4-Pyrimidyl | 5-O | |
| 3.130 | $CH_3$ | Cl | 2-SMe-6-Me-4-Pyrimidyl | 6-O | |
| 3.131 | $CH_3$ | Br | 2-S-Pr-4-Pyrimidyl | 5-O | |
| 3.132 | $CH_3$ | Cl | 2-Cl-4-Pyrimidyl | 6-S | |
| 3.133 | $CH_3$ | 1 | 2-SMe-6-Cl-4-Pyrimidyl | 5-O | |
| 3.134 | $CH_3$ | Cl | 5-MeO-3-Pyridazinyl | 6-O | |
| 3.135 | $CH_3$ | Br | 2-$CH_2$-5-Pyrimidyl | 5-O | |
| 3.136 | $CH_3$ | Cl | 4-Cl-5-Pyrimidyl | 6-S | |
| 3.137 | $CH_3$ | $CH_3$ | 2-SBu-5-Pyrimidyl | 6-O | |
| 3.138 | $CH_3$ | Br | 2-CN-4-$NO_2$-5-Pyrimidyl | 6-O | |
| 3.139 | $CH_3$ | $N(nC_4H_9)_2$ | 2-MeO-5-Pyrimidyl | 5-S | |
| 3.140 | $N(CH_3)_2$ | Br | 2-COOMe-5-Pyrimidyl | 5-S | |
| 3.141 | $CH_3$ | Cl | 4-$N(Bu)_2$-5-Pyrimidyl | 5-O | |
| 3.142 | $CH_3$ | Cl | 2,4-$Cl_2$-5-Pyrimidyl | 6-O | |
| 3.143 | $CH_3$ | $CF_3$ | 2,4-$(CH_3)_2$-5-Pyrimidyl | 5-S | |
| 3.144 | $CH_3$ | Br | 2-Pyrazinyl | 5-O | |
| 3.145 | $CH_3$ | Cl | 3-Chloro-2-Pyrazinyl | 6-O | |
| 3.146 | $CH_3$ | $OCF_3$ | 6-Chloro-2-Pyrazinyl | 5-S | |
| 3.147 | $CH_3$ | Cl | 3,6-$Me_2$-2-Pyrazinyl | 6-S | |
| 3.148 | $CH_3$ | Br | 6-COOMe-2-Pyrazinyl | 6-O | |
| 3.149 | $N(CH_3)_2$ | $CH_3$ | 3,5-$(NBu_2)_2$-6-CN-2-Pyrazinyl | 5-S | |
| 3.150 | Bu | $OCH_3$ | 3,5,6-$(CH_3)_3$-2-Pyrazinyl | 5-O | |
| 3.151 | $CH_3$ | Cl | 3-Et-2-Pyrazinyl | 6-O | |

| Verb. Nr. | $R_1$ | $R_2$ | $R_4$ | X | Smp. |
|---|---|---|---|---|---|
| 3.152 | $N(Et)_2$ | Cl | 5-OMe-2-Pyrazinyl | 6-S | |
| 3.153 | $CH_3$ | Br | 6-Br-2-Pyrazinyl | 5-O | |
| 3.154 | $CH_3$ | Br | 5-OMe-2-Pyrazinyl | 5-S | |
| 3.155 | $CH_3$ | Cl | 3-$NO_2$-2-Pyrazinyl | 6-S | |
| 3.156 | $CH_3$ | $OCF_2H$ | 3,5$(Cl)_2$-2-Pyridyl | 6-O | |
| 3.157 | $CH_3$ | Cl | 3,5$(Cl)_2$-2-Pyridyl | 5-S | |
| 3.158 | $CH_3$ | Cl | 3-Cl,5F-2-Pyridyl | 6-O | |
| 3.159 | $CH_3$ | Br | 3-Cl,5F-2-Pyridyl | 6-S | |
| 3.160 | $CH_3$ | F | 3-Cl,5$(CF_3)$-2-Pyridyl | 5-S | |
| 3.161 | $CH_3$ | Cl | 3-Cl,5$(CF_3)$-2-Pyridyl | 5-O | |
| 3.162 | $CH_3$ | Br | 3-Cl,5$(CF_3)$-2-Pyridyl | 6-O | 159-160°C |
| 3.163 | $CH_3$ | Cl | 4,6$(OCH_3)_2$-2-Pyrimidyl | 6-O | |
| 3.164 | $CH_3$ | Br | 4,6$(OCH_3)_2$-2-Pyrimidyl | 6-S | |
| 3.165 | $CH_3$ | Br | 5-Cl-6-Et-4-Pyrimidyl | 6-O | 164-165°C |
| 3.166 | $CH_3$ | Cl | 3-Cl-5-$CF_3$-2-Pyridyl | 6-O | 157-160°C |
| 3.167 | $N(CH_3)_2$ | Cl | 5-$CF_3$-2-Pyridyl | 6-O | 153-155°C |
| 3.168 | $N(CH_3)_2$ | Cl | 3-Cl-5-$CF_3$-2-Pyridyl | 6-O | 137-143°C |
| 3.169 | $CH_3$ | Br | 3-F-5-Cl-2-Pyridyl | 6-O | 178-179°C |
| 3.170 | $N(CH_3)_2$ | Br | 3-F-5-Cl-2-Pyridyl | 6-O | 148-149°C |
| 3.171 | $CH_3$ | Br | 6-$OCH_3$-3-Pyridazinyl | 6-O | 187-188°C |
| 3.172 | $CH_3$ | Br | 5-COOEt-2-Pyridyl | 6-O | 199-201°C |
| 3.173 | $CH_3$ | Br | 3-$CH_3$-2-Pyrimidyl | 6-O | 192-193°C |
| 3.174 | $N(CH_3)_2$ | Br | 3-$CH_3$-2-Pyrimidyl | 6-O | 179-181°C |
| 3.175 | $CH_3$ | Br | 2,6-$(OCH_3)_2$-4-Pyrimidyl | 6-O | |
| 3.176 | $CH_3$ | Br | 3-Ethyl-2-Pyrazinyl | 6-O | |
| 3.177 | $CH_3$ | Br | 6-$CH_3$-3-Pyridazinyl | 6-O | |
| 3.178 | $CH_3$ | Br | 6-$OCH_3$-3-Pyridazinyl | 6-O | 187-188°C |
| 3.179 | $CH_3$ | Br | 6-Cl-3-Pyridazinyl | 6-O | |
| 3.180 | $CH_3$ | Br | 4,5-$Cl_2$-3-Pyridazinyl | 6-O | |
| 3.181 | $N(CH_3)_2$ | Br | 5-Cl-6-$CH_3$-4-Pyrimidyl | 6-O | 166-169°C |
| 3.182 | $CH_3$ | Br | 5-Cl-6-$CH_3$-4-Pyrimidyl | 6-O | 188-190°C |

Tabelle 4

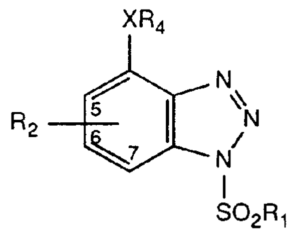

| Verb. Nr. | $R_1$ | $R_2$ | $R_4$ | X | Smp. |
|---|---|---|---|---|---|
| 4.1 | $CH_3$ | 5-H | 2-Pyridyl | S | |
| 4.2 | Et | 5-Cl | 2-Pyridyl | O | |
| 4.3 | s-$C_4H_9$ | 6-Br | 2-Pyridyl | O | |
| 4.4 | $N(CH_3)_2$ | 5-F | 2-Pyridyl | O | |
| 4.5 | $CH_3$ | 6-$CH_3$ | 3-Pyridyl | S | |
| 4.6 | $CH_3$ | 6-Cl | 4-Pyridyl | S | |
| 4.7 | $CH_3$ | 5-$CH_3O$ | 3-Cl-2-Pyridyl | S | |
| 4.8 | $CH_3$ | 6-$CF_3$ | 3-Cl-2-Pyridyl | O | |
| 4.9 | $CF_3$ | 5-Cl | 4-Cl-2-Pyridyl | S | |
| 4.10 | $CH_3$ | 6-Cl | 4-Cl-2-Pyridyl | S | |
| 4.11 | $N(CH_3)_2$ | 5-H | 5-Cl-2-Pyridyl | O | |
| 4.12 | $CH_3$ | 6-Br | 5-Cl-2-Pyridyl | O | |
| 4.13 | $CH_3$ | 6-$C_2H_5$ | 6-Cl-2-Pyridyl | S | |
| 4.14 | $CH_3$ | 5-n$C_4H_9$ | 4-Br-2-Pyridyl | O | |
| 4.15 | $CH_3$ | 6-$N(CH_3)_2$ | 4-Br-2-Pyridyl | O | |
| 4.16 | $N(CH_3)_2$ | 6-Cl | 3-F-2-Pyridyl | O | |
| 4.17 | $CH_3$ | 6-Br | 3-$CF_3$-2-Pyridyl | S | |
| 4.18 | $CH_3$ | 5-On$C_4H_9$ | 4-$CF_3$-2-Pyridyl | O | |
| 4.19 | $N(CH_3)_2$ | 6-F | 5-$CF_3$-2-Pyridyl | S | |
| 4.20 | $CH_3$ | 5-Cl | 5-$CF_3$-2-Pyridyl | S | |
| 4.21 | $CH_3$ | 5-H | 6-$CF_3$-2-Pyridyl | S | |
| 4.22 | $CH_3$ | 5-$OCF_2H$ | 3-$CH_3$-2-Pyridyl | O | |
| 4.23 | i-Pr | 6-Br | 3-$CH_3$-2-Pyridyl | S | |

| Verb. Nr. | $R_1$ | $R_2$ | $R_4$ | X | Smp. |
|---|---|---|---|---|---|
| 4.24 | $CH_3$ | 5-H | 3-$CH_3$-2-Pyridyl | O | |
| 4.25 | $CH_3$ | 6-n$C_4F_9$ | 4-$CH_3$-2-Pyridyl | S | |
| 4.26 | $CH_3$ | 6-Cl | 4-$CH_3$-2-Pyridyl | O | |
| 4.27 | n$C_4H_9$ | 6-Br | 5-$CH_3$-2-Pyridyl | O | |
| 4.28 | $CH_3$ | 6-H | 6-$CH_3$-2-Pyridyl | S | |
| 4.29 | $CH_3$ | 6-On$C_4H_9$ | 3-i-Pr-2-Pyridyl | O | |
| 4.30 | $CH_3$ | 6-N(n$C_4H_9$)$_2$ | 4-n-Hex-2-Pyridyl | S | |
| 4.31 | $CH_3$ | 5-F | 5-Et-2-Pyridyl | O | |
| 4.32 | $N(CH_3)_2$ | 6-H | 3-$OCH_2$-2-Pyridyl | O | |
| 4.33 | $CH_3$ | 5-Br | 4-$OCH_3$-2-Pyridyl | O | |
| 4.34 | $CH_3$ | 5-Cl | 5-OMe-2-Pyridyl | O | |
| 4.35 | $CH_3$ | 6-i$C_3H_7$ | 6-OMe-2-Pyridyl | O | |
| 4.36 | $CH_3$ | 6-$OC_2H_5$ | 4-$OEt_2$-2-Pyridyl | S | |
| 4.37 | $CH_3$ | 6-Br | 4-OiPr-2-Pyridyl | O | |
| 4.38 | $CH_3$ | 5-$OCF_3$ | 3-OnHex-2-Pyridyl | S | |
| 4.39 | $CH_3$ | 5-$CF_3$ | 5-OtBu--2-Pyridyl | S | |
| 4.40 | $CH_3$ | 6-Cl | 3-$OCF_3$-2-Pyridyl | O | |
| 4.41 | $CH_3$ | 6-Et | 4-$OCF_3$-2-Pyridyl | O | |
| 4.42 | $CH_3$ | 5-H | 5-$OCF_3$-2-Pyridyl | O | |
| 4.43 | $CH_3$ | 6-H | 6-$OCF_3$-2-Pyridyl | O | |
| 4.44 | $CH_3$ | 6-Otert.$C_4H_9$ | 3-$OCHF_2$-2-Pyridyl | S | |
| 4.45 | $CH_3$ | 6-H | 4-$OCHF_2$-2-Pyridyl | S | |
| 4.46 | $N(CH_3)_2$ | 6-Cl | 5-$OCHF_2$-2-Pyridyl | O | |
| 4.47 | $CH_3$ | 5-H | 6-$OCHF_2$-2-Pyridyl | O | |
| 4.48 | $CH_3$ | 6-Br | 6-$OCH_2CF_3$-2-Pyridyl | S | |
| 4.49 | $CH_3$ | 6-$C_2H_5$ | 6-$OC_6F_{13}$-2-Pyridyl | S | |
| 4.50 | $CH_3$ | 5-Oi$C_3H_7$ | 6-$NO_2$-2-Pyridyl | O | |
| 4.51 | $CH_3$ | 6-Br | 4-$NO_2$-2-Pyridyl | O | |
| 4.52 | Et | 5-Cl | 3-$NO_2$-2-Pyridyl | O | |
| 4.53 | $N(CH_3)_2$ | 5-H | 5-$NO_2$-2-Pyridyl | O | |
| 4.54 | $CH_3$ | 6-Br | 6-SMe-2-Pyridyl | S | |
| 4.55 | $CH_3$ | 5-Br | 6-S-Hexyl--2-Pyridyl | O | |

| Verb. Nr. | $R_1$ | $R_2$ | $R_4$ | X | Smp. |
|---|---|---|---|---|---|
| 4.56 | $CH_3$ | 5-H | 4-SMe-2-Pyridyl | S | |
| 4.57 | $CH_3$ | 6-$CF_3$ | 6-SiPr-2-Pyridyl | O | |
| 4.58 | $CH_3$ | 6-Cl | 3-COOMe-2-Pyridyl | O | |
| 4.59 | i-Pr | 5-H | 5-COOBu-2-Pyridyl | S | |
| 4.60 | $CH_3$ | 6-$CF_3$ | 5-COOMe-2-Pyridyl | S | |
| 4.61 | $CH_3$ | 6-Cl | 6-COOiPr-2-Pyridyl | O | |
| 4.62 | $CH_3$ | 6-$OiC_3H_7$ | 5-CN-2-Pyridyl | S | |
| 4.63 | $CH_3$ | 6-Cl | 6-CN-2-Pyridyl | O | |
| 4.64 | $CH_3$ | 5-Br | 4-$N(CH_3)_2$-2-Pyridyl | S | |
| 4.65 | $CH_3$ | 6-$OCF_3$ | 3-$N(Bu)_2$-2-Pyridyl | S | |
| 4.66 | $CH_3$ | 5-H | 6-$N(i\text{-}Pr)_2$-2-Pyridyl | O | |
| 4.67 | $CH_3$ | 6-$N(CH_3)_2$ | 4-$N(CH_3)(Et)$-2-Pyridyl | S | |
| 4.68 | $CH_3$ | 5-H | 3,5-$(Cl)_2$-2-Pyridyl | O | |
| 4.69 | $CH_3$ | 5-H | 3-Cl,5-F-2-Pyridyl | O | |
| 4.70 | $CH_3$ | 6-$OCH_3$ | 3,4,5,6$(Cl)_4$-2-Pyridyl | S | |
| 4.71 | $CH_3$ | 5-$CH_3$ | 3-$CF_3$, 5-Br-2-Pyridyl | O | |
| 4.72 | $CH_3$ | 5-H | 3-Cl, 5-$CF_3$-2-Pyridyl | O | |
| 4.73 | $CH_3$ | 6-$OiC_3H_7$ | 6-Cl-3-$CF_3$-2-Pyridyl | O | |
| 4.74 | $CH_3$ | 6-Cl | 6-Cl-4-$CF_3$-2-Pyridyl | S | |
| 4.75 | $CH_3$ | 5-H | 6-Cl-5-$CF_3$-2-Pyridyl | O | |
| 4.76 | nBu | 6-Br | 6-Br-4-$CF_3$-2-Pyridyl | S | |
| 4.77 | $CH_3$ | 5-$N(CH_3)_2$ | 4-Cl, 3-$CH_3$-2-Pyridyl | S | |
| 4.78 | $N(CH_3)_2$ | 5-$OCF_3$ | 6-Cl-5-$CH_3$-2-Pyridyl | O | |
| 4.79 | $CH_3$ | 6-$CH_3$ | 4-Br-5-$CH_3$-2-Pyridyl | S | |
| 4.80 | $CH_3$ | 6-Cl | 6-$CH_3$-4-$CF_3$-2-Pyridyl | O | |
| 4.81 | $CH_3$ | 5-H | 3-$CH_3$-4-Br-2-Pyridyl | S | |
| 4.82 | $CH_3$ | 6-Cl | 4,6-$(CH_3)_2$-3-CN-2-Pyridyl | S | |
| 4.83 | Et | 5-Cl | 3-COOMe-2-Pyridyl | O | |
| 4.84 | $CH_3$ | 5-H | 2-Br-3-Pyridyl | O | |
| 4.85 | $CH_3$ | 6-Br | 2-Cl-3-Pyridyl | O | |
| 4.86 | $CH_3$ | 6-$nC_4H_9$ | 2-Cl,6-$CH_3$-3-Pyridyl | S | |
| 4.87 | $CH_3$ | 5-$OCH_3$ | 5-Cl-3-Pyridyl | O | |

| Verb. Nr. | $R_1$ | $R_2$ | $R_4$ | X | Smp. |
|---|---|---|---|---|---|
| 4.88 | $CH_3$ | 6-Cl | 2,6($Br_2$)-3-Pyridyl | S | |
| 4.89 | $CH_3$ | 5-H | 6($CH_3$)-3-Pyridyl | O | |
| 4.90 | $CH_3$ | 5-Cl | 2-$NO_2$-3-Pyridyl | S | |
| 4.91 | $CH_3$ | 5-$iC_3H_7$ | 2-$NO_2$-6-$CH_3$-3-Pyridyl | S | |
| 4.92 | $CH_3$ | 6-Cl | 2-[$N(CH_3)_2$]-3-Pyridyl | O | |
| 4.93 | $CH_3$ | 6-$nC_4F_9$ | 2-COOMe-3-Pyridyl | S | |
| 4.94 | $CH_3$ | 6-Cl | 5-$CF_3$-3-Pyridyl | S | |
| 4.95 | $CH_3$ | 5-Br | 2,3,5,6-($Cl_4$)-4-Pyridyl | O | |
| 4.96 | $N(CH_3)_2$ | 6-Cl | 3,5-$Cl_2$-4-Pyridyl | S | |
| 4.97 | $CH_3$ | 5-$CH_3$ | 2-$CH_3$-4-Pyridyl | O | |
| 4.98 | $CH_3$ | 6-Br | 2-COOMe-4-Pyridyl | O | |
| 4.99 | Et | 6-sek.$C_4H_9$ | 2-Br-4-Pyridyl | S | |
| 4.100 | $N(CH_3)_2$ | 5-F | 2-Cl-4-Pyridyl | O | |
| 4.101 | $CH_3$ | 5-H | 2-$CF_3$-4-Pyridyl | O | |
| 4.102 | $CH_3$ | 5-H | 2-Pyrimidyl | O | |
| 4.103 | $CH_3$ | 6-$CF_3$ | 4-Pyrimidyl | O | |
| 4.104 | $CH_3$ | 5-$OCH_3$ | 5-Pyrimidyl | O | |
| 4.105 | $CH_3$ | 5-Br | 4-$CH_3$-2-Pyrimidyl | S | |
| 4.106 | $CH_3$ | 6-Cl | 4,6-($CH_3$)$_2$-2-Pyrimidyl | O | |
| 4.107 | $N(CH_3)_2$ | 5-H | 4,6-($CH_3$)$_2$-2-Pyrimidyl | S | |
| 4.108 | $CH_3$ | 6-$OiC_3H_7$ | 4-Cl-2-Pyrimidyl | O | |
| 4.109 | $CH_3$ | 6-Cl | 4,6(Cl)$_2$-2-Pyrimidyl | O | |
| 4.110 | $CH_3$ | 5-H | 4,5,6(Cl)$_3$-2-Pyrimidyl | O | |
| 4.111 | $CH_3$ | 6-$CF_3$ | 4-Cl-6-$CH_3$-2-Pyrimidyl | S | |
| 4.112 | $CH_3$ | 6-Br | 4-$CF_3$-5,6-$F_2$-2-Pyrimidyl | O | |
| 4.113 | $CH_3$ | 6-Cl | 5-Br-3-Pyridazinyl | O | |
| 4.114 | $CH_3$ | 6-Cl | 4-($CH_3$)-6($OCH_3$)-2-Pyrimidyl | S | |
| 4.115 | $N(CH_3)_2$ | 5-Cl | 4,6-$F_2$-2-Pyrimidyl | O | |
| 4.116 | $CH_3$ | 6-Br | 4-($CF_3$)-5-COOMe-2-Pyrimidyl | O | |
| 4.117 | $CH_3$ | 5-$N(CH_3)_2$ | 4-N(Bu)$_2$-5-F-2-Pyrimidyl | O | |
| 4.118 | $CH_3$ | 5-H | 4-$OC_4F_9$-2-Pyrimidyl | O | |
| 4.119 | $CH_3$ | 5-Cl | 4-(Cl)-5-($CH_3$)-2-Pyrimidyl | S | |

| Verb. Nr. | $R_1$ | $R_2$ | $R_4$ | X | Smp. |
|---|---|---|---|---|---|
| 4.120 | $CH_3$ | 5-H | 4,6-$(OMe)_2$-2-Pyrimidyl | O | |
| 4.121 | $CH_3$ | 5-H | 2,6-$Cl_2$-1-Pyrimidyl | S | |
| 4.122 | Bu | 6-F | 2-Cl,6-$CH_3$-4-Pyrimidyl | S | |
| 4.123 | $CH_3$ | 6-Br | 2-Cl-5-$CH_3$-4-Pyrimidyl | S | |
| 4.124 | $CH_3$ | 5-Cl | 5-Cl-6-Et-4-Pyrimidyl | O | |
| 4.125 | $CH_3$ | 5-H | 2,6-$(MeO)_2$-4-Pyrimidyl | S | |
| 4.126 | i-Pr | 6-Cl | 2-Cl,5-F-4-Pyrimidyl | O | |
| 4.127 | $CH_3$ | 5-$OCF_3$ | 2,5-$F_2$-6-$CF_3$-4-Pyrimidyl | O | |
| 4.128 | $CH_3$ | 5-$iC_3H_7$ | 2-Cl-6-$N(CH_3)_2$-4-Pyrimidyl | S | |
| 4.129 | $CH_3$ | 6-Br | 2,6-$F_2$-4-Pyrimidyl | O | |
| 4.130 | $CH_3$ | 5-Cl | 2-SMe-6-Me-4-Pyrimidyl | S | |
| 4.131 | $CH_3$ | 5-H | 2-S-Pr-4-Pyrimidyl | O | |
| 4.132 | $CH_3$ | 6-Cl | 2-Cl-4-Pyrimidyl | O | |
| 4.133 | $CH_3$ | 5-$OCH_3$ | 2-SMe-6-Cl-4-Pyrimidyl | S | |
| 4.134 | Bu | 5-$OnC_3H_7$ | 6-SMe-4-Pyrimidyl | O | |
| 4.135 | $CH_3$ | 5-H | 2-$CH_2$-5-Pyrimidyl | S | |
| 4.136 | $CH_3$ | 6-Cl | 4-Cl-5-Pyrimidyl | O | |
| 4.137 | $CH_3$ | 5-$CH_3$ | 2-SBu-5-Pyrimidyl | O | |
| 4.138 | $CH_3$ | 6-Br | 2-CN-4-$NO_2$-5-Pyrimidyl | S | |
| 4.139 | $CH_3$ | 6-$N(CH_3)_2$ | 2-MeO-5-Pyrimidyl | O | |
| 4.140 | $N(CH_3)_2$ | 5-H | 2-COOMe-5-Pyrimidyl | S | |
| 4.141 | $CH_3$ | 6-Cl | 4-$N(Bu)_2$-5-Pyrimidyl | O | |
| 4.142 | $CH_3$ | 6-Cl | 2,4-$Cl_2$-5-Pyrimidyl | O | |
| 4.143 | $CH_3$ | 6-$CF_3$ | 2,4-$(CH_3)_2$-5-Pyrimidyl | S | |
| 4.144 | Bu | 5-H | 2-Pyrazinyl | O | |
| 4.145 | $CH_3$ | 5-Cl | 3-Chloro-2-Pyrazinyl | S | |
| 4.146 | $CH_3$ | 5-Br | 6-Chloro-2-Pyrazinyl | S | |
| 4.147 | $CH_3$ | 6-Cl | 3,6-$Me_2$-2-Pyrazinyl | S | |
| 4.148 | $CH_3$ | 5-$CF_3$ | 3-Pyridazinyl | O | |
| 4.149 | $CH_3$ | 6-Br | 3,5-$(NBu_2)_2$-6-CN-2-Pyrazinyl | S | |
| 4.150 | $CH_3$ | 6-Cl | 3,5,6-$(CH_3)_3$-2-Pyrazinyl | O | |
| 4.151 | $CH_3$ | 5-Br | 3-Et-2-Pyrazinyl | O | |

Bu = n-$C_4H_9$

| Verb. Nr. | $R_1$ | $R_2$ | $R_4$ | X | Smp. |
|---|---|---|---|---|---|
| 4.152 | $CH_3$ | 6-Cl | 5-OMe-2-Pyrazinyl | O | |
| 4.153 | $CH_3$ | 6-Br | 6-Br-2-Pyrazinyl | S | |
| 4.154 | $CH_3$ | 5-H | 5-OMe-2-Pyrazinyl | O | |
| 4.155 | $N(CH_3)_2$ | 5-Cl | 3-$NO_2$-2-Pyrazinyl | O | |
| 4.156 | $CH_3$ | 5-Br | 3,5$(Cl)_2$-2-Pyridyl | S | |
| 4.157 | $CH_3$ | 6-Cl | 3,5$(Cl)_2$-2-Pyridyl | O | |
| 4.158 | $CH_3$ | 6-Cl | 3-Cl,5F-2-Pyridyl | O | |
| 4.159 | $N(CH_3)_2$ | 6-Br | 3-Cl,5F-2-Pyridyl | S | |
| 4.160 | $CH_3$ | 5-F | 3-Cl,5$(CF_3)$-2-Pyridyl | O | |
| 4.161 | $CH_3$ | 6-Cl | 3-Cl,5$(CF_3)$-2-Pyridyl | S | |
| 4.162 | $CH_3$ | 5-Br | 3-Cl,5$(CF_3)$-2-Pyridyl | O | |
| 4.163 | $CH_3$ | 6-Cl | 4,6$(OCH_3)_2$-2-Pyrimidyl | S | |
| 4.164 | $CH_3$ | 6-Br | 4,6$(OCH_3)_2$-2-Pyrimidyl | O | |

Formulierungsbeispiele für Wirkstoffe der Formel I (% = Gewichtsprozent)

| 2.1. Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.2. Emulsions-Konzentrat | |
|---|---|
| Wirkstoff aus den Tabellen | 10 % |
| Octylphenolpolyethylenglykolether | 3 % |
| (4-5 Mol Ethylenoxid) | |
| Ca-Dodecylbenzolsulfonat | 3 % |
| Ricinusölpolyglykolether | 4 % |
| (35 Mol Ethylenoxid) | |
| Cyclohexanon | 34 % |
| Xylolgemisch | 50 % |

Aus diesem Konzentrat können durch Verdünnen mit Wasser Emulsionen jeder gewünschten Konzentration hergestellt werden.

| 2.3. Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

| 2.4. Extruder Granulat | |
|---|---|
| Wirkstoff aus den Tabellen | 10 % |
| N-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| <u>2.5. Umhüllungs-Granulat</u> | |
|---|---|
| Wirkstoff aus den Tabellen | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.6. Suspensions-Konzentrat | |
|---|---|
| Wirkstoff aus den Tabellen | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung Silikonöl in Form einer 75%igen | 0,2 % |
| wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

3. Biologische Beispiele

Beispiel 3.1: Wirkung gegen Plasmopara viticola auf Reben

a) Residual-protektive Wirkung

Im 4-5 Blattstadium werden Rebensämlinge mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Nach einer Inkubation während 6 Tagen bei 95-100 % relativer Luftfeuchtigkeit und 20°C wird der Pilzbefall beurteilt.

b) Residual-kurative Wirkung

Im 4-5 Blattstadium werden Rebensämlinge mit einer Sporangiensuspension des Pilzes infiziert. Nach einer Inkubation während 24 Stunden in einer Feuchtkammer bei 95-100 % relativer Luftfeuchtigkeit und 20°C werden die infizierten Pflanzen getrocknet und mit einer aus Spritzpulver des Wirkstoffes hergestellten Spritzbrühe (0,02 % Aktivsubstanz) besprüht. Nach dem Antrocknen des Spritzbelages werden die behandelten Pflanzen wieder in die Feuchtkammer gebracht. Die Beurteilung des Pilzbefalls erfolgt 6 Tage nach der Infektion.

Verbindungen aus den Tabellen zeigen gegen Plasmopara viticola auf Reben eine sehr gute fungizide Wirkung, insbesondere die Wirkstoffe Nr. 1.20, 1.68, und 1.125 bewirken eine vollständige Unterdrückung des Pilzbefalls (Restbefall 0 bis 5 %). Unbehandelte aber infizierte Kontrollpflanzen weisen dagegen einen Plasmopara-Befall von 100 % auf.

Beispiel 3.2: Wirkung gegen Phytophthora auf Tomatenpflanzen

a) Residual-protektive Wirkung

Tomatenpflanzen werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90- 100 % relativer Luftfeuchtigkeit und 20°C.

b) Systemische Wirkung

Zu Tomatenpflanzen wird nach dreiwöchiger Anzucht eine aus Spritzpulver des Wirkstoffes hergestellte Spritzbrühe (0,002 % Aktivsubstanz bezogen auf das Erdvolumen) gegeben. Es wird dabei darauf geachtet, dass die Spritzbrühe nicht mit den oberirdischen Pflanzenteilen in Berührung kommt. Nach 48 Stunden werden

die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Verbindungen aus den Tabellen zeigen eine nachhaltige Wirkung (weniger als 20 % Pilzbefall). Mit den Verbindungen Nr. 1.20, 1.68 und 1.156 wird ein Befall praktisch vollständig verhindert (0 bis 5 % Befall). Unbehandelte aber infizierte Kontrollpflanzen weisen dagegen einen Phytophthora-Befall von 100 % auf.

Beispiel 3.3: Wirkung gegen Phytophthora auf Kartoffelpflanzen

a) Residual-protektive Wirkung

2-3 Wochen alte Kartoffelpflanzen (Sorte Bintje) werden nach 3-wöchiger Anzucht mit einer aus Spritzpulver des Wirkstoffes hergestellten Spritzbrühe (0,02 % Aktivsubstanz) besprüht. Nach 24 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

b) Systemische Wirkung

Zu 2-3 Wochen alten Kartoffelpflanzen (Sorte Bintje) wird nach dreiwöchiger Anzucht eine aus Spritzpulver des Wirkstoffes hergestellte Spritzbrühe (0,002 % Aktivsubstanz bezogen auf das Erdvolumen) gegeben. Es wird dabei darauf geachtet, dass die Spritzbrühe nicht mit den oberirdischen Pflanzenteilen in Berührung kommt. Nach 48 Stunden werden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20°C.

Verbindungen aus den Tabellen zeigen eine nachhaltige Wirkung (weniger als 20 % Pilzbefall). Mit den Verbindungen Nr. 1.20, 1.68 und 1.156 wird ein Befall praktisch vollständig verhindert (0 bis 5 % Befall). Unbehandelte aber infizierte Kontrollpflanzen weisen dagegen einen Phytophthora-Befall von 100 % auf.

## Patentansprüche

1. Benztriazolsulfonsäure-Derivate der Formel I

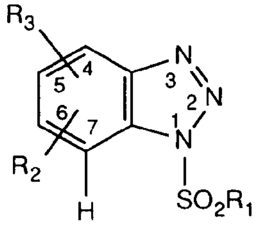

(I)

worin die $R_1SO_2$-Gruppe die 1- oder 3-Position besetzt und im Verhältnis zu den Substituenten $R_3$ und $R_2$ reine oder gemischte Stellungsisomere bildet, und worin die Substituenten folgende Bedeutung haben:

| | |
|---|---|
| $R_3$ = | $R_4X$ |
| X = | Sauerstoff oder Schwefel; |
| $R_4$ = | ungesättigter 6-gliedriger Heterocyclus mit maximal zwei N-Atomen, der unsubstituiert oder durch mindestens einen der Substituenten Halogen, $C_1$-$C_6$-Alkyl, $C_1$-$C_6$-Alkoxy, $C_1$-$C_6$-Alkylthio, $C_1$-$C_6$-Haloalkyl, $C_1$-$C_6$-Haloalkoxy, $C_3$-$C_6$-Cycloalkyl, Cyano, Nitro, -COO($C_1$-$C_6$-Alkyl) und N(R')(R") substituiert sein kann; |
| $R_2$ = | Wasserstoff, Halogen, $C_1$-$C_4$-Alkyl, $C_1$-$C_4$-Alkoxy, $C_1$-$C_4$-Haloalkyl, $C_1$-$C_4$-Haloalkoxy und -N(alk)$_2$; |
| $R_1$ = | $C_1$-$C_4$-Alkyl, $C_1$-$C_4$-Haloalkyl, $C_3$-$C_6$-Cycloalkyl, oder -N(alk)$_2$, wobei alk $C_1$-$C_4$-Alkyl bedeutet und in gleicher oder verschiedener Bedeutung an N gebunden ist; |
| R'und R" = | unabhängig Wasserstoff oder $C_1$-$C_4$-Alkyl. |

2. Benztriazolsulfonsäure-Derivate der Formel I, gemäss Anspruch 1, worin die $R_1SO_2$-Gruppe die 1- oder

3-Position besetzt und im Verhältnis zu den Substituenten $R_3$ und $R_2$ reine oder gemischte Stellungsisomere bildet, und worin die Substituenten folgende Bedeutung haben:

| | |
|---|---|
| $R_3$ = | $R_4X$ |
| X = | Sauerstoff oder Schwefel; |
| $R_4$ = | ungesättigter 6-gliedriger Heterocyclus mit maximal zwei N-Atomen, der unsubstituiert oder durch mindestens einen der Substituenten Halogen, $C_1$-$C_6$-Alkyl, $C_1$-$C_6$-Alkoxy, $C_1$-$C_6$-Alkylthio, $C_1$-$C_6$-Haloalkyl, $C_1$-$C_6$-Haloalkoxy, Cyano, Nitro, -COO($C_1$-$C_6$-Alkyl) und N(R')(R") substituiert sein kann; |
| $R_2$ = | Wasserstoff, Halogen, $C_1$-$C_4$-Alkyl, $C_1$-$C_4$-Alkoxy, $C_1$-$C_4$-Haloalkyl, $C_1$-$C_4$-Haloalkoxy und -N(alk)$_2$; |
| $R_1$ = | $C_1$-$C_4$-Alkyl, $C_1$-$C_4$-Haloalkyl, $C_3$-$C_6$-Cycloalkyl, oder -N(alk)$_2$, wobei alk $C_1$-$C_4$-Alkyl bedeutet und in gleicher oder verschiedener Bedeutung an N gebunden ist; |
| R' und R" = | unabhängig Wasserstoff oder $C_1$-$C_4$-Alkyl. |

**3.** Verbindungen gemäss Anspruch 2, worin $R_4$ einen substituerten Pyridin-, Pyrimidin-, Pyrazin- oder Pyridazinring bedeutet.

**4.** Verbindungen gemäss Anspruch 1, worin der 6-gliedrige Heterocyclus Pyridin-, Pyrimidin-, Pyrazin- oder Pyridazinring bedeutet durch ein bis drei Substituenten ausgewählt aus Halogen, Methyl, Ethyl, Isopropyl, Methoxy, $C_1$-$C_2$-Haloalkyl mit F und/oder Cl als Halogen, $CF_3O$, $CHF_2O$, Cyclopropyl, Nitro, substituiert ist.

**5.** Verbindungen gemäss Anspruch 3, worin der 6-gliedrige Heterocyclus durch ein bis drei Substituenten ausgewählt aus Halogen, Methyl, Ethyl, Isopropyl, Methoxy, $C_1$-$C_2$-Haloalkyl mit F und/oder Cl als Halogen, $CF_3O$, $CHF_2O$, Nitro, substituiert ist.

**6.** Verbindungen gemäss Anspruch 2, worin der 6-gliedrige Heterocyclus unsubstituiertes oder ein- bis dreifach substituiertes Pyridin ist und $R_2$ Wasserstoff, Fluor, Chlor, Brom, Methyl, Methoxy, $CF_3$, $CF_3O$ oder $CHF_2O$ bedeutet.

**7.** Verbindungen gemäss Anspruch 6, worin $R_2$ Wasserstoff, Chlor oder Brom ist.

**8.** Verbindungen gemäss Anspruch 1, worin $R_4$ Pyridin ist und der Pyridinring durch maximal drei Substituenten ausgewählt aus Halogen, $C_1$-$C_4$-Alkyl, $C_1$-$C_4$-Alkoxy, $C_1$-$C_4$-Haloalkyl, $C_1$-$C_4$-Haloalkoxy und Cyclopropyl substituiert ist.

**9.** Verbindungen gemäss Anspruch 6, worin der Pyridinring durch maximal drei Substituenten ausgewählt aus Halogen, $C_1$-$C_4$-Alkyl, $C_1$-$C_4$-Alkoxy, $C_1$-$C_4$-Haloalkyl und $C_1$-$C_4$-Haloalkoxy substituiert ist.

**10.** Verbindungen gemäss Anspruch 9, worin der Pyridinring mindestens durch $CF_3$ substituiert ist.

**11.** Verbindungen gemäss Anspruch 2, worin der 6-gliedrige Heterocyclus unsubstituiertes oder ein- bis dreifach substituiertes Pyrimidin ist und $R_2$ Wasserstoff, Fluor, Chlor, Brom, Methyl, Methoxy, $CF_3$, $CF_3O$ oder $CHF_2O$ bedeutet.

**12.** Verbindungen gemäss Anspruch 11, worin $R_2$ Wasserstoff, Chlor oder Brom ist.

**13.** Verbindungen gemäss Anspruch 11, worin der Pyrimidinring durch maximal drei Substituenten ausgewählt aus Halogen, $C_1$-$C_4$-Alkyl, $C_1$-$C_4$-Alkoxy, $C_1$-$C_4$-Haloalkyl und $C_1$-$C_4$-Haloalkoxy substituiert ist.

**14.** Verbindungen gemäss einem der Ansprüche 2 bis 13, worin $R_1$ Methyl bedeutet.

**15.** Verbindungen gemäss einem der Ansprüche 2 bis 14, worin die 4-Position des Benztriazolrings unsubstituiert ist.

**16.** Verfahren zur Herstellung von Verbindungen der Formel I gekennzeichnet
1) durch Reaktion einer Verbindung der Formel II

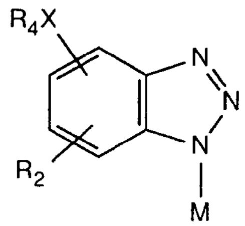

(II)

einer Verbindung der Formel III

$Q\text{-}SO_2\text{-}R_1$ (III)

worin X, $R_1$, $R_2$ und $R_4$ die unter Formel I angegebenen Bedeutungen besitzen und M Wasserstoff oder ein Alkalimetall, bevorzugt Na, K oder Li, und Q ein Halogenatom, bevorzugt Chlor, oder den Rest O-$SO_2$-$R_1$ darstellen, in einem inerten Lösungsmittel, in Gegenwart oder Abwesenheit einer Base, bei -30°C bis +180; oder

2) durch Diazotierung einer Verbindung der Formel IV

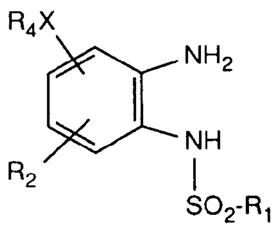

(IV)

worin X, $R_1$, $R_2$ und $R_4$ die unter Formel I angegebenen Bedeutungen besitzen, entweder

a) mit einem anorganischen Nitrit in einem Lösungsmittel, bevorzugt in Wasser oder in Gemischen von Wasser mit Alkoholen, bevorzugt Methanol oder Ethanol, oder in Gemischen von Wasser mit Ether, bevorzugt Dioxan oder Dimethoxyethan, in Gegenwart einer Säure bevorzugt Essigsäure bei Temperaturen von -30° bis +180°C; oder

b) mit einem organischen Nitrit, bevorzugt Ethylnitrit, Amylnitrit oder tert.Butylnitrit, in einem Lösungsmittel, bevorzugt Alkohole wie Methanol oder Ethanol oder Ether wie Dioxan oder Dimethoxyethan, gegebenenfalls in Gegenwart einer Säure, bei Temperaturen von -30° bis + 180°C

**17.** Mittel zur Bekämpfung und Verhütung eines Befalls von Pflanzen durch Mikroorganismen dadurch gekennzeichnet, dass es als Wirkstoff eine Verbindung der Formel I gemäss Anspruch 1, 4 oder 8 zusammen mit einem geeigneten Trägermaterial enthält.

**18.** Mittel gemäss Anspruch 17, wobei der Wirkstoff eine der Verbindungen gemäss einem der Ansprüche 2, 3, 5 bis 7 und 9 bis 15 ist.

**19.** Verfahren zur Bekämpfung und Verhütung eines Befalls von Pflanzen durch Mikroorganismen, dadurch gekennzeichnet, dass man eine Verbindung der Formel I gemäss Anspruch 1, 4 oder 8 auf die Pflanze, auf Pflanzenteile oder auf den Nährboden der Pflanzen appliziert.

**20.** Verfahren gemäss Anspruch 19, wobei eine Verbindung gemäss einem der Ansprüche 2, 3, 5 bis 7 und 9 bis 15 verwendet wird.

Europäisches Patentamt

# EUROPÄISCHER RECHERCHENBERICHT

Nummer der Anmeldung

EP 92 81 0995

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,A | US-A-2 943 017 (K. SASSE ET AL.)<br>* das ganze Dokument *<br>--- | 1-20 | C07D401/12<br>C07D403/12<br>A01N43/647 |
| P,X | EP-A-0 462 931 (CIBA-GEIGY AG)<br>* das ganze Dokument *<br>----- | 1-20 | |

RECHERCHIERTE SACHGEBIETE (Int. Cl.5 )

C07D

Der vorliegende Recherchenbericht wurde für alle Patentansprüche erstellt

| Recherchenort | Abschlußdatum der Recherche | Prüfer |
|---|---|---|
| DEN HAAG | 18 MAERZ 1993 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)